# EUROPEAN PATENT SPECIFICATION

(11) **EP 2 287 332 B1**
(45) Date of publication and mention of the grant of the patent: **10.12.2014**
(21) Application number: 09010238.5
(22) Date of filing: 07.08.2009
(51) Int. Cl.: C12Q 1/68

(54) **Nucleic acids and methods for environmental monitoring**
Nukleinsäuren und Verfahren zur Umweltüberwachung
Acides nucléiques et procédé de surveillance environnementale

(43) Date of publication of application: 23.02.2011
(73) Proprietor: Biotecon Diagnostics GmbH, 14473 Postdam (DE)
(72) Inventor: Berghof, Kornelia, 12355 Berlin (DE); Grönewald, Astrid, 13353 Berlin (DE)
(74) Representative: Grünecker, Kinkeldey, Stockmair & Schwanhäusser

(56) References cited:
- US-A1- 2003 082 656
- US-B1- 6 194 145
- US-B1- 7 002 005
- US-B1- 7 202 027
- DATABASE Geneseq [Online] 18 October 2007 (2007-10-18), "Human protease/osteoarthritis gene-specific probe - SEQ ID 228490." XP002550414 retrieved from EBI accession no. GSN:AFX03066 Database accession no. AFX03066
- DATABASE Geneseq [Online] 18 October 2007 (2007-10-18), "Human protease/osteoarthritis gene-specific probe - SEQ ID 189484." XP002550415 retrieved from EBI accession no. GSN:AFW64059 Database accession no. AFW64059
- REGENSBURGER A ET AL: "DNA PROBES WITH DIFFERENT SPECIFICITIES FROM A CLONED 23S RIBOSOMAL RNA GENE OF MICROCOCCUS-LUTEUS" JOURNAL OF GENERAL MICROBIOLOGY, vol. 134, no. 5, 1988, pages 1197-1204, XP002550413 ISSN: 0022-1287

### SEQUENCE LISTING

<110> BIOTECON Diagnostics GmbH
<120> Nucleic Acids and Method for Environmental Monitoring
<130> EP66514
<160> 108
<170> PatentIn version 3.3
<210> 1
   <211> 18
   <212> DNA
   <213> Artificial
<220>
   <223> primer or probe
<400> 1
   cgtgagacag ttcggtct 18
<210> 2
   <211> 19
   <212> DNA
   <213> Artificial
<220>
   <223> primer or probe
<400> 2
   gacgaacctc tggtatgtc 19
<210> 3
   <211> 17
   <212> DNA
   <213> Artificial
<220>
   <223> primer or probe
<400> 3
   ggctggttgg ctacgta 17
<210> 4
   <211> 17
   <212> DNA
   <213> Artificial
<220>
   <223> primer or probe
<400> 4
   ggctggttgg ctatgtg 17
<210> 5
   <211> 19
   <212> DNA
   <213> Artificial
<220>
   <223> primer or probe
<400> 5
   gattagctac gttcgggat 19
<210> 6
   <211> 20
   <212> DNA
   <213> Artificial
<220>
   <223> primer or probe
<400> 6
   attagctacg ttcggaagag 20
<210> 7
   <211> 20
   <212> DNA
   <213> Artificial
<220>
   <223> primer or probe
<400> 7
   attagctacg ttcggaagtg 20
<210> 8
   <211> 16
   <212> DNA
   <213> Artificial
<220>
   <223> primer or probe
<400> 8
   ggctacgttc gggaga 16
<210> 9
   <211> 17
   <212> DNA
   <213> Artificial
<220>
   <223> primer or probe
<400> 9
   tcgctacgtt cggaaga 17
<210> 10
   <211> 23
   <212> DNA
   <213> Artificial
<220>
   <223> primer or probe
<400> 10
   ccgctgaaag catctaagcg gga 23
<210> 11
   <211> 23
   <212> DNA
   <213> Artificial
<220>
   <223> primer or probe
<400> 11
   ccgctgaaag catctaagtg gga 23
<210> 12
   <211> 23
   <212> DNA
   <213> Artificial
<220>
   <223> primer or probe
<400> 12
   ccgctgaagg catctaagcg gga 23
<210> 13
   <211> 23
   <212> DNA
   <213> Artificial
<220>
   <223> primer or probe
<400> 13
   cccgcttaga tgctttcagc ggt 23
<210> 14
   <211> 36
   <212> DNA
   <213> Artificial
<220>
   <223> primer or probe
<400> 14
   gggracctca aawgaaacct catctyaaaa caggct 36
<210> 15
   <211> 33
   <212> DNA
   <213> Artificial
<220>
   <223> primer or probe
<400> 15
   cctgttttaa gatgaggttt cgttaaggtc ccc 33
<210> 16
   <211> 34
   <212> DNA
   <213> Artificial
<220>
   <223> primer or probe
<400> 16
   cctgttttaa gatgaggttt catttgaggt bccc 34
<210> 17
   <211> 34
   <212> DNA
   <213> Artificial
<220>
   <223> primer or probe
<400> 17
   cctgttycra gatgaggttt cwtttgaggt tccc 34
<210> 18
   <211> 34
   <212> DNA
   <213> Artificial
<220>
   <223> primer or probe
<400> 18
   cctgttktga gatgaggttt cktttgaggt tccc 34
<210> 19
   <211> 31
   <212> DNA
   <213> Artificial
<220>
   <223> primer or probe
<400> 19
   cctgtttcaa gatgaggttt cataggttcc c 31
<210> 20
   <211> 30
   <212> DNA
   <213> Artificial
<220>
   <223> primer or probe
<400> 20
   cctgttttga gatgaggttt cttaggcgcc 30
<210> 21
   <211> 27
   <212> DNA
   <213> Artificial
<220>
   <223> primer or probe
<400> 21
   ttaggggacc ttaacgaaac ctcatct 27
<210> 22
   <211> 30
   <212> DNA
   <213> Artificial
<220>
   <223> primer or probe
<400> 22
   atagggaacc tcaaaagaaa cctcatctca 30
<210> 23
   <211> 28
   <212> DNA
   <213> Artificial
<220>
   <223> primer or probe
<400> 23
   cctgggaacc tcaaatgaaa cctcatct 28
<210> 24
   <211> 27
   <212> DNA
   <213> Artificial
<220>
   <223> primer or probe
<400> 24
   tgggggacct caaatgaaac ctcatct 27
<210> 25
   <211> 34
   <212> DNA
   <213> Artificial
<220>
   <223> primer or probe
<400> 25
   agggracctc aaaygaaacc tcatctyrra acag 34
<210> 26
   <211> 17
   <212> DNA
   <213> Artificial
<220>
   <223> primer or probe
<400> 26
   tctaagcggg aagcttg 17
<210> 27
   <211> 16
   <212> DNA
   <213> Artificial
<220>
   <223> primer or probe
<400> 27
   cggcttcgag atgaga 16
<210> 28
   <211> 17
   <212> DNA
   <213> Artificial
<220>
   <223> primer or probe
<400> 28
   tttccttgcc cctttga 17
<210> 29
   <211> 18
   <212> DNA
   <213> Artificial
<220>
   <223> primer or probe
<400> 29
   tctccttgcc cttttgtt 18
<210> 30
   <211> 24
   <212> DNA
   <213> Artificial
<220>
   <223> primer or probe
<400> 30
   ggaaatctca tctcgaagcc ggct 24
<210> 31
   <211> 25
   <212> DNA
   <213> Artificial
<220>
   <223> primer or probe
<400> 31
   aggagaactc atctcgaagc aagct 25
<210> 32
   <211> 25
   <212> DNA
   <213> Artificial
<220>
   <223> primer or probe
<400> 32
   aggaaatctc atctcggagc aagct 25
<210> 33
   <211> 26
   <212> DNA
   <213> Artificial
<220>
   <223> primer or probe
<400> 33
   ctggaaatct catctcgaag cgagct 26
<210> 34
   <211> 24
   <212> DNA
   <213> Artificial
<220>
   <223> primer or probe
<400> 34
   ggaaacctca tcttgaagcg tgct 24
<210> 35
   <211> 30
   <212> DNA
   <213> Artificial
<220>
   <223> primer or probe
<400> 35
   gcgggaagct tgcttcgaga tgagatttcc 30
<210> 36
   <211> 25
   <212> DNA
   <213> Artificial
<220>
   <223> primer or probe
<400> 36
   cttgcttcga gatgagttct ccttg 25
<210> 37
   <211> 28
   <212> DNA
   <213> Artificial
<220>
   <223> primer or probe
<400> 37
   cttgctccga gatgagattt ccttcccc 28
<210> 38
   <211> 26
   <212> DNA
   <213> Artificial
<220>
   <223> primer or probe
<400> 38
   tcgcttcgag atgagatttc cagaca 26
<210> 39
   <211> 26
   <212> DNA
   <213> Artificial
<220>
   <223> primer or probe
<400> 39
   ccggcttcga gatgagattt ccttgc 26
<210> 40
   <211> 26
   <212> DNA
   <213> Artificial
<220>
   <223> primer or probe
<400> 40
   cacgcttcaa gatgaggttt ccatgc 26
<210> 41
   <211> 22
   <212> DNA
   <213> Artificial
<220>
   <223> primer or probe
<400> 41
   tgcccctttg agggtgtgag gc 22
<210> 42
   <211> 21
   <212> DNA
   <213> Artificial
<220>
   <223> primer or probe
<400> 42
   tgcccttttg ttgggtgtga g 21
<210> 43
   <211> 25
   <212> DNA
   <213> Artificial
<220>
   <223> primer or probe
<400> 43
   agacaccatt tggtgtgaga ggccc 25
<210> 44
   <211> 26
   <212> DNA
   <213> Artificial
<220>
   <223> primer or probe
<400> 44
   taatcccttt tgtgggggtg tgaggt 26
<210> 45
   <211> 21
   <212> DNA
   <213> Artificial
<220>
   <223> primer or probe
<400> 45
   aacccccgta agggggtgtc a 21
<210> 46
   <211> 21
   <212> DNA
   <213> Artificial
<220>
   <223> primer or probe
<400> 46
   ccataatctc tagggaacct c 21
<210> 47
   <211> 32
   <212> DNA
   <213> Artificial
<220>
   <223> primer or probe
<400> 47
   cttccagrtc yggcctatca accccatmgt ct 32
<210> 48
   <211> 32
   <212> DNA
   <213> Artificial
<220>
   <223> primer or probe
<400> 48
   cttmcagatc yggcctatca accccatmat ct 32
<210> 49
   <211> 32
   <212> DNA
   <213> Artificial
<220>
   <223> primer or probe
<400> 49
   cttccagrtc cggcctatca acccagtmgt ct 32
<210> 50
   <211> 26
   <212> DNA
   <213> Artificial
<220>
   <223> primer or probe
<400> 50
   aggtctggcc tatctaccct atcgtc 26
<210> 51
   <211> 16
   <212> DNA
   <213> Artificial
<220>
   <223> primer or probe
<400> 51
   ggcccccagc tagaac 16
<210> 52
   <211> 18
   <212> DNA
   <213> Artificial
<220>
   <223> primer or probe
<400> 52
   ggctccctgt agatgacg 18
<210> 53
   <211> 21
   <212> DNA
   <213> Artificial
<220>
   <223> primer or probe
<400> 53
   gcctatcaac ccagtagtct a 21
<210> 54
   <211> 19
   <212> DNA
   <213> Artificial
<220>
   <223> primer or probe
<400> 54
   ctatcaaccc catcgtctc 19
<210> 55
   <211> 19
   <212> DNA
   <213> Artificial
<220>
   <223> primer or probe
<400> 55
   aacactgggt tgataggct 19
<210> 56
   <211> 26
   <212> DNA
   <213> Artificial
<220>
   <223> primer or probe
<400> 56
   ccagcctatc aacccagtgt tctagc 26
<210> 57
   <211> 21
   <212> DNA
   <213> Artificial
<220>
   <223> primer or probe
<400> 57
   accccgtcat ctacagggag c 21
<210> 58
   <211> 18
   <212> DNA
   <213> Artificial
<220>
   <223> primer or probe
<400> 58
   atgtggaagc gaggactg 18
<210> 59
   <211> 23
   <212> DNA
   <213> Artificial
<220>
   <223> primer or probe
<400> 59
   agtcttcagt cctcgcttcc aca 23
<210> 60
   <211> 23
   <212> DNA
   <213> Artificial
<220>
   <223> primer or probe
<400> 60
   ccggcttcca tacctggcct atc 23
<210> 61
   <211> 23
   <212> DNA
   <213> Artificial
<220>
   <223> primer or probe
<400> 61
   cgtcttcagt ccgtgcttcc aca 23
<210> 62
   <211> 28
   <212> DNA
   <213> Artificial
<220>
   <223> primer or probe
<400> 62
   ccacacccgg cctatcaacc cagtagtc 28
<210> 63
   <211> 23
   <212> DNA
   <213> Artificial
<220> ,
   <223> primer or probe
<400> 63
   cagcttcacg agtcttcagt cct 23
<210> 64
   <211> 17
   <212> DNA
   <213> Artificial
<220>
   <223> primer or probe
<400> 64
   gctccacgcc ttacaac 17
<210> 65
   <211> 21
   <212> DNA
   <213> Artificial
<220>
   <223> primer or probe
<400> 65
   cacacgtcgt tggttcgtgc t 21
<210> 66
   <211> 20
   <212> DNA
   <213> Artificial
<220>
   <223> primer or probe
<400> 66
   acgagtcgtc agtcctcgct 20
<210> 67
   <211> 24
   <212> DNA
   <213> Artificial
<220>
   <223> primer or probe
<400> 67
   ggtcatatta gtatcggtca tctc 24
<210> 68
   <211> 24
   <212> DNA
   <213> Artificial
<220>
   <223> primer or probe
<400> 68
   gttttggtct attagtacca gtag 24
<210> 69
   <211> 22
   <212> DNA
   <213> Artificial
<220>
   <223> primer or probe
<400> 69
   gtggtcaatt agtaccagta gc 22
<210> 70
   <211> 23
   <212> DNA
   <213> Artificial
<220>
   <223> primer or probe
<400> 70
   gttggtgtat tagtaccagt cac 23
<210> 71
   <211> 22
   <212> DNA
   <213> Artificial
<220>
   <223> primer or probe
<400> 71
   tcggtaaatt agtaccagtc ac 22
<210> 72
   <211> 20
   <212> DNA
   <213> Artificial
<220>
   <223> primer or probe
<400> 72
   ggccaattag taccagtcac 20
<210> 73
   <211> 20
   <212> DNA
   <213> Artificial
<220>
   <223> primer or probe
<400> 73
   ggtcaattag taccggtcac 20
<210> 74
   <211> 20
   <212> DNA
   <213> Artificial
<220>
   <223> primer or probe
<400> 74
   ggctaattag taccggtcac 20
<210> 75
   <211> 19
   <212> DNA
   <213> Artificial
<220>
   <223> primer or probe
<400> 75
   ggtgtattag taccggtcg 19
<210> 76
   <211> 22
   <212> DNA
   <213> Artificial
<220>
   <223> primer or probe
<400> 76
   aagtcatcgg cctattagta ct 22
<210> 77
   <211> 23
   <212> DNA
   <213> Artificial
<220>
   <223> primer or probe
<400> 77
   aagtcttcgg tgtattagta cca 23
<210> 78
   <211> 24
   <212> DNA
   <213> Artificial
<220>
   <223> primer or probe
<400> 78
   gaagatcatc ggcttattag tact 24
<210> 79
   <211> 17
   <212> DNA
   <213> Artificial
<220>
   <223> primer or probe
<400> 79
   ccagtcaact ccaccag 17
<210> 80
   <211> 21
   <212> DNA
   <213> Artificial
<220>
   <223> primer or probe
<400> 80
   cttattagta ccggtcagct c 21
<210> 81
   <211> 15
   <212> DNA
   <213> Artificial
<220>
   <223> primer or probe
<400> 81
   agtaccggtc ggctg 15
<210> 82
   <211> 20
   <212> DNA
   <213> Artificial
<220>
   <223> primer or probe
<400> 82
   caactccacc agtcttcagt 20
<210> 83
   <211> 19
   <212> DNA
   <213> Artificial
<220>
   <223> primer or probe
<400> 83
   acaccaacac acgaccatc 19
<210> 84
   <211> 19
   <212> DNA
   <213> Artificial
<220>
   <223> primer or probe
<400> 84
   ggctgcaaga catgttgtg 19
<210> 85
   <211> 17
   <212> DNA
   <213> Artificial
<220>
   <223> primer or probe
<400> 85
   acgcgaacac acagatc 17
<210> 86
   <211> 21
   <212> DNA
   <213> Artificial
<220>
   <223> primer or probe
<400> 86
   tcagacaacc gcacagtgga c 21
<210> 87
   <211> 17
   <212> DNA
   <213> Artificial
<220>
   <223> primer or probe
<400> 87
   agtggacgcg aaacacg 17
<210> 88
   <211> 20
   <212> DNA
   <213> Artificial
<220>
   <223> primer or probe
<400> 88
   ttgctatccc aaaaccatac 20
<210> 89
   <211> 17
   <212> DNA
   <213> Artificial
<220>
   <223> primer or probe
<400> 89
   ccccttcgct atgacca 17
<210> 90
   <211> 17
   <212> DNA
   <213> Artificial
<220>
   <223> primer or probe
<400> 90
   gcttagcttc cgggttc 17
<210> 91
   <211> 18
   <212> DNA
   <213> Artificial
<220>
   <223> primer or probe
<400> 91
   ctactctccc acaccctc 18
<210> 92
   <211> 23
   <212> DNA
   <213> Artificial
<220>
   <223> primer or probe
<400> 92
   gcagtaccat cagcgctaag agc 23
<210> 93
   <211> 20
   <212> DNA
   <213> Artificial
<220>
   <223> primer or probe
<400> 93
   gatttcccaa cttcggttat 20
<210> 94
   <211> 20
   <212> DNA
   <213> Artificial
<220>
   <223> primer or probe
<400> 94
   gaacgtaagt ccgactacca 20
<210> 95
   <211> 21
   <212> DNA
   <213> Artificial
<220>
   <223> primer or probe
<400> 95
   cgtaagttcg actaccatcg a 21
<210> 96
   <211> 19
   <212> DNA
   <213> Artificial
<220>
   <223> primer or probe
<400> 96
   aacgtcagtc cgactacca 19
<210> 97
   <211> 21
   <212> DNA
   <213> Artificial
<220>
   <223> primer or probe
<400> 97
   ggaacgtaaa tccaactacc a 21
<210> 98
   <211> 18
   <212> DNA
   <213> Artificial
<220>
   <223> primer or probe
<400> 98
   acgtaagccc aactacca 18
<210> 99
   <211> 21
   <212> DNA
   <213> Artificial
<220>
   <223> primer or probe
<400> 99
   gggatgtaaa tccaactacc a 21
<210> 100
   <211> 20
   <212> DNA
   <213> Artificial
<220>
   <223> primer or probe
<400> 100
   ggatgtaagt ccgactacca 20
<210> 101
   <211> 21
   <212> DNA
   <213> Artificial
<220>
   <223> primer or probe
<400> 101
   ggattgtaag cccaactact a 21
<210> 102
   <211> 24
   <212> DNA
   <213> Artificial
<220>
   <223> primer or probe
<400> 102
   agcaaagagg tcacacctgt tccc 24
<210> 103
   <211> 24
   <212> DNA
   <213> Artificial
<220>
   <223> primer or probe
<400> 103
   agcaaggagg tcacacctgt tccc 24
<210> 104
   <211> 27
   <212> DNA
   <213> Artificial
<220>
   <223> primer or probe
<400> 104
   tgccgaacac agaagttaag ctcctta 27
<210> 105
   <211> 27
   <212> DNA
   <213> Artificial
<220>
   <223> primer or probe
<400> 105
   tgccgaacac agtagttaag ctcttta 27
<210> 106
   <211> 20
   <212> DNA
   <213> Artificial
<220>
   <223> primer or probe
<400> 106
   cgatcgatta gtattcgtca 20
<210> 107
   <211> 17
   <212> DNA
   <213> Artificial
<220>
   <223> primer or probe
<400> 107
   tccacatgtc accatgc 17
<210> 108
   <211> 28
   <212> DNA
   <213> Artificial
<220>
   <223> primer or probe
<400> 108
   cattatccga tacgttcttc cagtgcca 28

## Description

### Background of the Invention

Environmental monitoring is an important part of the manufacture of sterile products, especially in the aseptic production of pharmaceuticals, but also for the production of non-sterile products. The current good manufacturing practise (cGMP) requires that the quality of a pharmaceutical product does not just occur through the control of the finished product, but also has to be assured through a controlled process. Taking samples for sterility testing is only significant for the whole production-lot if the microbiological status of the environment is tested at the same time. These environmental tests have to be carried out regularly by monitoring the working area as well as the personnel and by using appropriate controls. A functional environmental monitoring process is a prerequisite for the release of parenteral pharmaceuticals.

The requirements regarding the environmental monitoring depend on the risk that the environment would pose to the product quality. The production facilities are therefore divided into defined critical and controlled areas with different demands on testing intervals and warning and action levels concerning total microbial counts. Sampling methods vary for air, surfaces, and personnel, but all of them amount to the detection of microbial growth occurring on solid media (*i.e*., agar plates) after an incubation period. If colonies are present on the plates, they are then further analyzed and identified at an appropriate level in order to determine the typical flora of the respective environment. Identification to a certain degree is also useful in order to draw conclusions as to the source of the contamination and to possible influence factors.

A study conducted in a pharmaceutical production facility (Hitschfeld 2005) lists the most common bacteria found during environmental monitoring. The vast majority (80 % - 90 %) were Gram-positive bacteria belonging mainly to the genera *Micrococcus, Corynebacterium,* and *Staphylococcus,* with *Micrococcus luteus* being the most frequent species (> 25 % for personnel, > 34 % for air, > 23 % for surfaces). The remaining microorganisms cannot be assigned to such groups but are phylogenetically diverse and were mostly identified less than four times in one year. While these microorganisms constitute the variable flora; the Gram-positive bacteria with high GC-content as well as staphylococci represent the typical baseline flora. A more or less constant percentage of these organisms indicates that the environment is operating within an adequate state of control.

It is an aspect of the present invention to provide nucleic acids which discriminate between the typical baseline flora and the variable flora.

It is another aspect of the present invention to provide nucleic acids which discriminate between the most frequent species in environmental monitoring, *Micrococcus luteus,* and the rest of the baseline flora.

### State of the Art

Identification of the organisms that grow on agar plates sampled in the process of environmental monitoring is usually performed with conventional phenotypical methods such as: (i) Gram stain, followed by (ii) microscopy to read the Gram stain reaction and determine the cellular morphology of the isolate, and depending on the results, (iii) biochemical tests. Throughout the different steps of theses conventional methods which in many cases can take days, secondary contaminations often occur. It is almost impossible to notice with these conventional methods when a bacterial colony is overgrown by another organism with similar characteristics.

Gram stain and microscopy are critical steps for the identification process as their results usually determine which further biochemical tests are conducted. The outcome of a Gram stain can vary with the age of bacterial colonies, and with the quality of the reagents used for the test. Cell morphology may also vary with colony age, and even for the experienced microbiologist it is difficult to distinguish between short rods and large cocci under the microscope.

Biochemical tests usually rely on the direct or indirect detection of enzymes expressed by the microorganisms and therefore require freshly and densely grown pure cultures of single organisms. In stressed microorganisms (*e.g.*, by temperature or selective growth media) the expression of the enzymes may be partly or fully inhibited. It is also well known to those experienced in the art that biochemical characteristics of a species only apply to the majority of isolates belonging to this species while there are always exceptional strains (Bergey 2001). Various studies have shown the limitations of commercial biochemical test systems concerning specificity (Brigante et al. 2008; Delmas et al. 2008).

Since the shortcomings of phenotypic methods are well known there is a growing demand for genotypical methods. From 2004 on, the use of more sensitive and specific tests has been recommended by the European Pharmacopoeia (E.P., Chapters 5.1.6 and 2.6.1) and by the FDA Aseptic Guideline with reference to molecular biological methods.

Identification methods based on nucleic acid analyses include DNA-DNA hybridization, polymerase chain reaction (PCR), and sequencing. In particular, sequencing of the 16S rDNA region has become a valuable tool in bacterial taxonomy and identification. However, the generation of templates for sequencing, the sequencing reaction itself, and the analysis of the sequencing results (usually a comparison with a database of reference sequences) is a time-consuming, expensive, and potentially error-prone process.

The application of PCR systems in the pharmaceutical industry has concentrated on the detection of pathogens, as the demonstration of the absence of pathogens is an important step in the release testing process (Merker et al. 2000).

Thus, there is a need in the art for a simple and easy to handle method which enables the rapid and inexpensive detection and classification of bacteria constituting the normal environmental background flora.

### Embodiments of the invention are:

1. A kit comprising a combination of nucleic acid molecules suitable in the detection of the taxonomic unit comprising or consisting of the genera *Micrococcus, Kocuria, Kytococcus, Nesterenkonia,* the combination comprising
   (i)
      (a) the combination of SEQ ID NOs: 2, 7 and 9;
      (b) the combination of a nucleic acid molecule which hybridizes under stringent conditions with SEQ ID NO: 2, a nucleic acid molecule which hybridizes under stringent conditions with SEQ ID NO: 7 and a nucleic acid molecule which hybridizes under stringent conditions with SEQ ID NO: 9;
      (c) the combination of a nucleic acid molecule which is at least 90 % identical with SEQ ID NO: 2, a nucleic acid molecule which is at least 90 % identical with SEQ ID NO:7, a nucleic acid molecule which is at least 90 % identical with SEQ ID NO: 9;or
      (d) the combination of the complements of the nucleic acid molecules of (i) ((a), (b) or (c); together with
   (ii)
      (a) the combination of SEQ ID NOs: 64, 79, 81, 86, and 87;
      (b) the combination of a nucleic acid molecule which hybridizes under stringent conditions with SEQ ID NO: 64, a nucleic acid molecule which hybridizes under stringent conditions with SEQ ID NO: 79, a nucleic acid molecule which hybridizes under stringent conditions with SEQ ID NO: 81, a nucleic acid molecule which hybridizes under stringent conditions with SEQ ID NO: 86, , and a nucleic acid molecule which hybridizes under stringent conditions with SEQ ID NO: 87;
      (c) the combination of a nucleic acid molecule which is at least 90 % identical with SEQ ID NO: 64, a nucleic acid molecule which is at least 90 % identical with SEQ ID NO:79, a nucleic acid molecule which is at least 90 % identical with SEQ ID NO: 81, a nucleic acid molecule which is at least 90 % identical with SEQ ID NO:86, and a nucleic acid molecule which is at least 90 % identical with SEQ ID NO: 87; or
      (d) the combination of the complements of the nucleic acid molecules of (ii) (a), (b) or (c)
2. The kit of embodiment 1, further comprising a nucleic acid selected from
   (a) SEQ ID NOs: 10, 11, 12, 36, 37, 38, 39 or 40;
   (b) a nucleic acid molecule which hybridizes under stringent conditions with a nucleic acid molecule of (a);
   (c) a nucleic acid molecule which is at least 90 % identical with the nucleic acid molecule of (a); or
   (d) the complement of a nucleic acid molecule of (a) to (c).
3. The kit of any of embodiments 1 to 2, which is also suitable for use in the detection of the genus *Corynebacterium,* the combination further comprising
   i) SEQ ID NO: 1; or a variant thereof; and
      one of SEQ ID NOs: 67, 68, 69, 70, 71, 73, 75; or a respective variant of one of these; or
   ii) SEQ ID NOs: 3 and 4 or a respective variant of one of these; and
      one of the SEQ ID NOs: 46, 90 or 91; or a respective variant of one of these,
   wherein a variant of a nucleic acid molecule of i) or ii) is
   a) a nucleic acid molecule which hybridizes under stringent conditions with the nucleic acid molecule;
   b) a nucleic acid molecule which is at least 90 % identical with the nucleic acid molecule of a); or
   c) the complement of the nucleic acid molecule or of one of the variants a) to b).
4. The kit of any of embodiments 1 to 4, which is also suitable for use in the detection of the genus *Staphylococcus,* the combination further comprising
   i) SEQ ID NO: 93; or a variant thereof; and
      one of SEQ ID NOs: 94-101; or a respective variant of one of these;
   wherein a variant of a nucleic acid molecule of i) is
   a) a nucleic acid molecule which hybridizes under stringent conditions with the nucleic acid molecule;
   b) a nucleic acid molecule which is at least 90 % identical with the nucleic acid molecules of a); or
   c) the complement of the nucleic acid molecule or of one of the variants a) to b).
5. A method for amplifying or identifying bacterial DNA of the taxonomic unit comprising or consisting of the genera *Micrococcus, Kocuria, Kytococcus, Nesterenkonia* in a sample, in which
   a) in a first amplification step the DNA of the taxonomic unit is amplified with conserved primers, wherein the primers used in the first amplification step comprise all of the nucleic acids as defined in embodiment 1 (i) (a) or all of the nucleic acids as defined in embodiment 1 (i) (b) or all of the nucleic acids as defined in embodiment 1 (i) (c); together with all of the nucleic acids as defined in embodiment 1 (ii) (a) or all of the nucleic acids as defined in embodiment 1 (ii) (b) or all of the nucleic acids as defined in embodiment 1 (ii) (c).
6. The method of embodiment 5, wherein
   b) in a further detection step, the DNA fragments obtained by amplification step a) are detected by means of probes,
   wherein the probes used in step b) comprise one or more of the nucleic acids as defined in embodiment 2 (a) to (d).
7. The method of any of embodiments 5 or 6, which in the same sample also allows for amplifying or identifying the genus *Corynebacterium* and/or *Staphylococcus,* in which method
   i) in a second amplification step, which is simultaneous or subsequent to the first amplification step, the DNA of the genus *Corynebacterium* is amplified with conserved primers, and/or
   ii) in a third amplification step, which is simultaneous or subsequent to the first amplification step, the DNA of the genus *Staphylococcus* is amplified with conserved primers.
8. The method of any of embodiments 5 to 7, wherein the primers used in the second amplification step comprise one or more of the nucleic acids selected from
   a) SEQ ID NOs: 1, 3 or 4,
   b) SEQ ID NOs: 46, 90, 91, 67, 68, 69, 70, 71, 73, 75, 72 or 74;
   c) a nucleic acid molecule which hybridizes under stringent conditions with the nucleic acid molecule of any of a) to b);
   d) a nucleic acid molecule which is at least 90 % identical with the nucleic acid molecule of a), or b); or
   e) the complement of the nucleic acid molecule of any of a) or b).
9. The method of any of embodiments 5 to 8, wherein the primers used in the third amplification step comprise one or more of the nucleic acids selected from
   a) SEQ ID NO: 93;
   b) SEQ ID NOs: 94, 95, 96, 97, 98, 99, 100 or 101;
   c) a nucleic acid molecule which hybridizes under stringent conditions with the nucleic acid molecule of any of a) to b);
   d) a nucleic acid molecule which is at least 90 % identical with the nucleic acid molecule of a), or b); or
   e) the complement of the nucleic acid molecule of any of a) or b).
10. The method of any of embodiments 5 to 9, further allowing the amplification or detection of one or more of the genera *Micrococcus, Kocuria, Kytocoxus* and/or *Nesterenkonia* or one or more species within one or more of these genera.
11. The method of any of embodiments 5 to 10, further allowing the amplification or detection of one or more of *Micrococcus luteus, Staphylococcus aureus* or *Dermacoccus nishinomiyaensis.*

### Description of the Invention

The present invention provides nucleic acids and methods for a rapid classification and identification of predetermined groups of bacteria typically occurring in the environment and on human skin and are therefore part of the baseline flora found in environmental monitoring.

Organisms not being a member of the predetermined groups are preferably not identified.

The term predetermined group is used to describe a part or the whole of the bacteria topically occurring in the environment and on human skin. The members of the group can be chosen from one genus, but can also be chosen from different genera.

One preferred group consists of the genera *Corynebacterium, Micrococcus, Kocuria, Kytococcus,* and *Nesterenkonla.* Another preferable group consists of the genus *Corynebactenum.* Another preferable group consists of the species *Micrococcus luteus.*

The present invention provides inter alia a reliable and specific means for the detection of microorganisms of the taxonomic unit set forth by the genera *Micrococcus, Kocuria, Kytococcus, Nesterenkonla,* which optionally also allows for further determination and discrimination of other microorganisms of other taxonomic units and of one or more genera or species within the genera *Micrococcus, Kocuria, Kytococcus, Nesterenkonla.*

The inventors have found that oligonucleotides or combinations thereof targeting the genomic region from the 3'-end of the 23S-rDNA to the 3'-end of the 5S-rDNA gene including the internal transcribed spacer region (ITS 2) enable such a specific detection of the typical environmental monitoring flora. They also discovered oligonucleotides targeting this region that allow a specific detection of the most frequent species found on environmental monitoring plates, *i.e. Micrococcus luteus.*

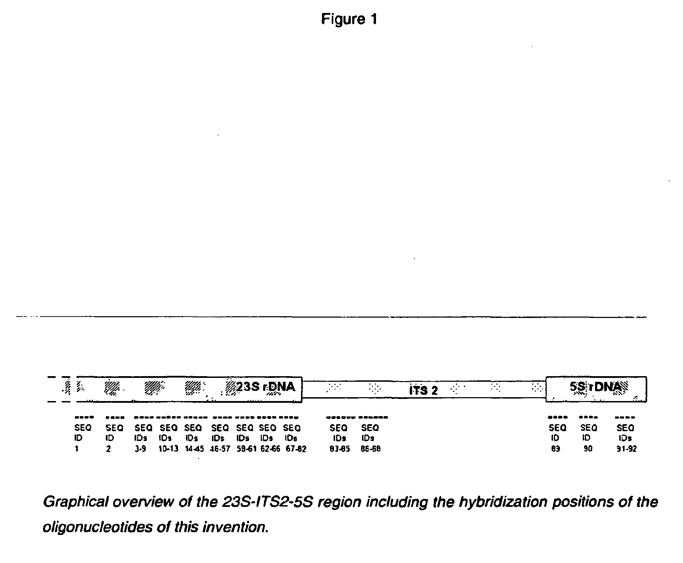

Figure 1 shows the DNA region as well as the hybridization positions of the oligonucleotides of this invention.

The design of specific oligonucleotides involved identification of sequences which could serve as target sites, *i.e.* which have high sequence homologies within the predetermined group of organisms and low homologies for organisms not being members of the groups. For this purpose, sequence alignments were developed, and a number of nucleic acids were designed based on these analyses.

The Sequences cited in the claims of the present invention are shown in Table 1.

**Table 1:**

| **SEQ ID No.** | **Sequence 5' - 3'** |
|---|---|
| SEO ID No. 1 | CGTGAGACAGTTCGGTCT |
| SEQ ID No. 2 | GACGAACCTCTGGTATGTC |
| SEQ ID No. 3 | GGCTGGTTGGCTACGTA |
| SEQ ID No. 4 | GGCTGGTTGGCTATGTG |
| SEQ ID No. 5 | GATTAGCTACGTTCGGGAT |
| SEQ ID No. 6 | ATTAGCTACGTTCGGAAGAG |
| SEQ ID No. 7 | ATTAGCTACGTTCGGAAGTG |
| SEQ ID No. 8 | GGCTACGTTCGGGAGA |
| SEQ ID No. 9 | TCGCTACGTTCGGAAGA |
| SEQ ID No. 10 | CCGCTGAAAGCATCTAAGCGGGA |
| SEO ID No. 11 | CCGCTGAAAGCATCTAAGTGGGA |
| SEQ ID No. 12 | CCGCTGAAGGCATCTAAGCGGGA |
| SEQ ID No. 13 | CCCGCTTAGATGCTTTCAGCGGT |
| SEQ ID No. 14 | GGGRACCTCAAAWGAAACCTCATCTYAAAACAGGCT |
| SEQ ID No. 15 | CCTGTTTTAAGATGAGGTTTCGTTAAGGTCCCC |
| SEQ ID No. 16 | CCTGTTTTAAGATGAGGTTTCATTTGAGGTBCCC |
| SEQ ID No. 17 | CCTGTTYCRAGATGAGGTTTCWTTTGAGGTTCCC |
| SEQ ID No. 18 | CCTGTTKTGAGATGAGGTTTCKTTTGAGGTTCCC |
| SEQ ID No. 19 | CCTGTTTCAAGATGAGGTTTCATAGGTTCCC |
| SEQ ID No. 20 | CCTGTTTTGAGATGAGGTTTCTTAGGCGCC |
| SEQ ID No. 21 | TTAGGGGACCTTAACGAAACCTCATCT |
| SEQ ID No. 22 | ATAGGGAACCTCAAAAGAAACCTCATCTCA |
| SEQ ID No. 23 | CCTGGGAACCTCAAATGAAACCTCATCT |
| SEQ ID No. 24 | TGGGGGACCTCAAATGAAACCTCATCT |
| SEQ ID No. 25 | AGGGRACCTCAAAYGAAACCTCATCTYRRAACAG |
| SEQ ID No. 26 | TCTAAGCGGGAAGCTTG |
| SEQ ID No. 27 | CGGCTTCGAGATGAGA |
| SEQ ID No. 28 | TTTCCTTGCCCCTTTGA |
| SEQ ID No. 29 | TCTCCTTGCCCTTTTGTT |
| SEQ ID No. 30 | GGAAATCTCATCTCGAAGCCGGCT |
| SEQ ID No. 31 | AGGAGAACTCATCTCGAAGCAAGCT |
| SEQ ID No. 32 | AGGAAATCTCATCTCGGAGCAAGCT |
| SEQ ID No. 33 | CTGGAAATCTCATCTCGAAGCGAGCT |
| SEQ ID No. 34 | GGAAACCTCATCTTGAAGCGTGCT |
| SEQ ID No. 35 | GCGGGAAGCTTGCTTCGAGATGAGATTTCC |
| SEQ ID No. 36 | CTTGCTTCGAGATGAGTTCTCCTTG |
| SEQ ID No. 37 | CTTGCTCCGAGATGAGATTTCCTTCCCC |
| SEQ ID No. 38 | TCGCTTCGAGATGAGATTTCCAGACA |
| SEQ ID No. 39 | CCGGCTTCGAGATGAGATTTCCTTGC |
| SEQ ID No. 40 | CACGCTTCAAGATGAGGTTTCCATGC |
| SEQ ID No. 41 | TGCCCCTTTGAGGGTGTGAGGC |
| SEQ ID No. 42 | TGCCCTTTTGTTGGGTGTGAG |
| SEQ ID No. 43 | AGACACCATTTGGTGTGAGAGGCCC |
| SEQ ID No. 44 | TAATCCCTTTTGTGGGGGTGTGAGGT |
| SEQ ID No. 45 | AACCCCCGTAAGGGGGTGTCA |
| SEQ ID No. 46 | CCATAATCTCTAGGGAACCTC |
| SEQ ID No. 47 | CTTCCAGRTCYGGCCTATCAACCCCATMGTCT |
| SEQ ID No. 48 | CTTMCAGATCYGGCCTATCAACCCCATMATCT |
| SEQ ID No. 49 | CTTCCAGRTCCGGCCTATCAACCCAGTMGTCT |
| SEQ ID No. 50 | AGGTCTGGCCTATCIACGGTATCGTC |
| SEQ ID No. 51 | GGCCCCCAGCTAGAAC |
| SEQ ID No. 52 | GGCTCCCTGTAGATGACG |
| SEQ ID No. 53 | GCCTATCAACCCAGTAGTCTA |
| SEQ ID No. 54 | CTATCAACCCCATCGTCTC |
| SEQ ID No. 55 | AACACTGGGTTGATAGGCT |
| SEQ ID No. 56 | CCAGCCTATCAACCCAGTGTTCTAGC |
| SEQ ID No. 57 | ACCCCGTCATCTACAGGGAGC |
| SEQ ID No. 58 | ATGTGGAAGCGAGGACTG |
| SEQ ID No. 59 | AGTCTTCAGTCCTCGCTTCCACA |
| SEQ ID No. 60 | CCGGCTTCCATACCTGGCCTATC |
| SEQ ID No. 61 | CGTCTTCAGTCCGTGCTTCCACA |
| SEQ ID No. 62 | CCACACCCGGCCTATCAACCCAGTAGTC |
| SEQ ID No. 63 | CAGCTTCACGAGTCTTCAGTCCT |
| SEO ID No. 64 | GCTCCACGCCTTACAAC |
| SEQ ID No. 65 | CACACGTCGTTGGTTCGTGCT |
| SEQ ID No. 66 | ACGAGTCGTCAGTCCTCGCT |
| SEQ ID No. 67 | GGTCATATTAGTATCGGTCATCTC |
| SEQ ID No. 68 | GTTTTGGTCTATTAGTACCAGTAG |
| SEQ ID No. 69 | GTGGTCAATTAGTACCAGTAGC |
| SEQ ID No. 70 | GTTGGTGTATTAGTACCAGTCAC |
| SEQ ID No. 71 | TCGGTAAATTAGTACCAGTCAC |
| SEQ ID No. 72 | GGCCAATTAGTACCAGTCAC |
| SEQ ID No. 73 | GGTCAATTAGTACCGGTCAC |
| SEQ ID No. 74 | GGCTAATTAGTACCGGTCAC |
| SEQ ID No. 75 | GGTGTATTAGTACCGGTCG |
| SEQ ID No. 76 | AAGTCATCGGCCTATTAGTACT |
| SEQ ID No. 77 | AAGTCTTCGGTGTATTAGTACCA |
| SEQ ID No. 78 | GAAGATCATCGGCTTATTAGTACT |
| SEQ ID No. 79 | CCAGTCAACTCCACCAG |
| SEQ ID No. 80 | CTTATTAGTACCGGTCAGCTC |
| SEQ ID No. 81 | AGTACCGGTCGGCTG |
| SEQ ID No. 82 | CAACTCCACCAGTCTTCAGT |
| SEQ ID No. 83 | ACACCAACACACGACCATC |
| SEQ ID No. 84 | GGCTGCAAGACATGTTGTG |
| SEQ ID No. 85 | ACGCGAACACACAGATC |
| SEQ ID No. 86 | TCAGACAACCGCACAGTGGAC |
| SEQ ID No. 87 | AGTGGACGCGAAACACG |
| SEQ ID No. 88 | TTGCTATCCCAAAACCATAC |
| SEQ ID No. 89 | CCCCTTCGCTATGACCA |
| SEQ ID No. 90 | GCTTAGCTTCCGGGTTC |
| SEQ ID No. 91 | CTACTCTCCCACACCCTC |
| SEQ ID No. 92 | GCAGTACCATCAGCGCTAAGAGC |
| SEQ ID No. 93 | GATTTCCCAACTTCGGTTAT |
| SEQ ID No. 94 | GAACGTAAGTCCGACTACCA |
| SEQ ID No. 95 | CGTAAGTTCGACTACCATCGA |
| SEQ ID No. 96 | AACGTCAGTCCGACTACCA |
| SEQ ID No. 97 | GGAACGTAAATCCAACTACCA |
| SEQ ID No. 98 | ACGTAAGCCCAACTACCA |
| SEQ ID No. 99 | GGGATGTAAATCCAACTACCA |
| SEQ ID No. 100 | GGATGTAAGTCCGACTACCA |
| SEQ ID No. 101 | GGATTGTAAGCCCAACTACTA |
| SEQ ID No. 102 | AGCAAAGAGGTCACACCTGTTCCC |
| SEQ ID No. 103 | AGCAAGGAGGTCACACCTGTTCCC |
| SEQ ID No. 104 | TGCCGAACACAGAAGTTAAGCTCCTTA |
| SEQ ID No. 105 | TGCCGAACACAGTAGTTAAGCTCTTTA |
| SEQ ID No. 106 | CGATCGATTAGTATTCGTCA |
| SEQ ID No. 107 | TCCACATGTCACCATGC |
| SEQ ID No. 108 | CATTATCCGATACGTTCTTCCAGTGCCA |

The specificity and usefulness of the oligonucleotides is set forth in table 2.

**Table 2:**

| ***"Micrococcus"* Group of Organisms including the genera *Micrococcus, Kocuria, Kytococcus, Nesterenkonia* (all formerly classified as *Micrococcus*)** | **SEQ IDs** |
|---|---|
| preferred primers | 2, 7, 9 / 64, 79, 81, 86, 87 |
| preferred probe pairs | 10 - 12 / 36 - 40 |

| ***Micrococcus* Genus** | **SEQ IDs** |
|---|---|
| preferred primers | 2 / 86 |
| further possible primers | 5 or 27 or 28 or 51 / 63 or 76 |
| preferred probe pairs | 10, 11 / 39 |
| further possible probe pairs | 30 / 13 |
| | 59, 61 / 56 |

| | 35 / 41 |
|---|---|
| probe pair for identification of *Micrococcus luteus* | 55 / 58 |
| ***Kocuria* species** | **SEQ IDs** |
| *Kocuria rosea* preferred primers | 9 / 81 |
| *Kocuria rosea* preferred probe pairs | 10 / 38 |
| *Kocuria rosea* further possible probe pairs | 33 / 13 |
| | 65 / 62 |
| | 35 / 43 |
| *Kocuria kristinae* preferred primers | 2 / 79 |
| *Kocuria kristinae* further possible primers | 8 or 26 or 29 or 52 / 82 or 83 or 88 or 92 |
| *Kocuria kristinae* preferred probe pairs | 10, 12 / 36 |
| *Kocuria kristinae* further possible probe pairs | 31 / 13 |
| | 60 / 57 |
| | 35 / 42 |
| *Kocuria palustris* preferred primers | 2 / 87 |
| *Kocuria palustris* further possible primers | 8 or 26 / 78 or 84 |
| *Kocuria palustris* preferred probe pairs | 10 / 37 |
| *Kocuria palustris* further possible probe pairs | 66 / 62 |
| *Kocuria carniphila* preferred primers | 2 / 64 |
| *Kocuria carniphila* further possible primers | 6 or 26 / 85 |
| *Kocuria carniphila* preferred probe pairs | 10 / 37 |
| *Kocuria carniphila* further possible probe pairs | 32 / 13 64 / 62 |
| *Kocuria varians* and *Kocuria rhizophila* preferred primers | 2 / 64 |
| *Kocuria varians* and *Kocuria rhizophila* further possible primers | 5 or 26 / 77 |
| *Kocuria varians* and *Kocuria rhizophila* preferred probe pairs | 10 / 37 |
| *Kocuria varians* and *Kocuria rhizophila* further possible probe pairs | 64 / 62 35 / 44, 45 |

| ***Kytococcus* Genus and *Nesterenkonia* Genus** | **SEQ IDs** |
|---|---|
| preferred primers | 7 / 86 |
| further possible primers | / 53 or 80 or 89 |
| preferred probe pairs | 10 / 40 |
| further possible probe pairs | 34 / 13 |

| ***Dermacoccus nishinomiyaensis* (formerly classified as *Micrococcus*)** | **SEQ IDs** |
|---|---|
| preferred primers | 7 / 54 |
| preferred probe pairs | 34 / 13 |

| ***Corynebacterium* Genus** | **SEQ IDs** |
|---|---|
| preferred primers | 1 / 67 - 71, 73, 75 |
| further possible primers | 3, 4 / 46 or 90 or 91 |
| preferred probe pairs | 10 / 15 - 20 |
| further possible probe pairs | 50 / 21 - 24 |
| | 47 - 49 / 25 |
| | 14 / 13 |

| ***Staphylococcus* Genus** | **SEQ IDs** |
|---|---|
| primers | 93 / 94 - 101 |
| probe pairs | 102, 103 / 104, 105 |
| probe pair for identification of *Staphylococcus aureus* | 106 / 107 |

| **Internal Amplification Control** | **SEQ IDs** |
|---|---|
| specific probe | 108 |

### Definitions

### Nucleotides

Nucleotides are the modules of the DNA or RNA. The following abbreviations are used: G=Guanosine, A=Adenosine, T=Thymidine, C=Cytidine, R=G or A, Y=C or T, K=G or T, W=A or T, S=C or G, M=A or C, B=C, G or T, D=A, G or T, H=A, C or T, V=A, C or G, N=A, C, G or T, I=Inosine.

### Nucleic Acid

A nucleic acid is a DNA, RNA, PNA, or LNA which is obtained either through isolation from genomic DNA or from cDNA according to known standard methods and purified or generated artificially using known methods such as oligonucleotide synthesis or isolated as ribosomal RNA or mRNA from the organism or synthesised as PNA or LNA.

A "PNA" is a peptide nucleic acid in which instead of the phosphoric acid backbone of the DNA, 2-aminoethylglycin compounds occur.

A "LNA" is a locked nucleic acid in which the pentose moiety of DNA or RNA is modified to contain a 2'-O, 4'-C methylene linkage (1,2:5,6-di-O-isopropylene-alpha-D-allofuranose) to "lock" the nucleic acid in a certain conformational state.

According to the invention, in the nucleic acids, up to 20 % of the nucleotides in 10 consecutive nucleotides, preferably 1 nucleotide from a block of 10 consecutive nucleotides may be replaced by nucleotides (*e.g*. inosin, etc.) which do not naturally occur in bacteria.

The nucleic acids may further contain modifications which allow the production of a signal that can be detected directly or indirectly. The expert is aware of the following modifications here:
(i) radioactive modifications, *i.e*. radioactive phosphorylation or radioactive labelling with sulphur, hydrogen, carbon, nitrogen;
(ii) coloured groups (*e.g*. digoxygenin, etc.);
(iii) fluorescent groups (*e.g*. fluorescein, etc.);
(iv) chemoluminescent groups;
(v) groups for the immobilisation at a solid phase (*e.g*. biotin, streptag, antibodies, antigens, etc.); and/or
(vi) groups which allow an indirect or direct reaction with the help of antibodies, antigens, enzymes and/or substances with an affinity to enzymes or enzyme complexes;
or combinations of modifications according to two or more of the modifications listed under (i) to (vi). The term "modification" as used in this invention is understood to mean directly or indirectly detectable groups or groups for immobilisation at a solid phase which are attached to the nucleic acid. Metal atoms, radioactive, coloured or fluorescent groups are directly detectable groups. Immunologically or enzymatically detectable groups are indirectly detectable groups, such as antigens and antibodies, haptenes or enzymes or parts of enzymes with an enzymatic effect. These indirect groups are detected in subsequent reactions. Preference is given to haptenes which are linked to an oligonucleotide and which are detected in a subsequent antibody reaction.

### Fragments of Oligonucleotides

Fragments of oligonucleotides arise due to deletion of one or more nucleotides on the 5' and/or 3' end of an oligonucleotide.

### Identical Sequences/Percentage of Identity

For the determination of the identity (in the sense of complete matching, corresponding to 100 % identity) of nucleic acid sequences, partial sequences of a larger polynucleotide are considered. These partial sequences comprise ten nucleotides and are then identical when all 10 modules are identical for two comparative sequences. The nucleotides thymidine and uridine are identical. As partial sequences, all possible fragments of a larger polynucleotide can be considered.

As an example two polynucleotides are considered which comprise 20 nucleotides and which differ in the 5th module. In a sequence comparison six 10-way nucleotides are found which are identical and five which are not identical, because they differ in one module.

In addition, the identity can be gradually determined, whereby the unit is stated in percent. For the determination of the degree of identity partial sequences are also considered, which comprise as a minimum the length of the actually used sequence, e.g. as primer, or 20 nucleotides.

As an example, polynucleotide A with a length of 100 nucleotides and B with a length of 200 nucleotides are compared. A primer with a length of 14 nucleotides is derived from polynucleotide B. For the determination of the degree of identity, polynucleotide A is compared with the primer over its complete length. If the sequence of the primer occurs in polynucleotide A, whereby it however deviates in one module, then there is a fragment with a degree of identity of 13:14, *i.e*. approx. 93%.

In the second example the polynucleotides A and B previously mentioned are compared in their entirety. In this case all the possible comparative windows of a length of 20 nucleotides are applied and the degree of identity determined for them. If then nucleotides nos. 50-69 of polynucleotide A and B are identical with the exception of nucleotide no. 55, then a degree of identity of 19:20, *i.e.* 95% arises for these fragments.

### Primer

Primers are oligonucleotides which act as starter molecules during a PCR. Here, they hybridize on a target molecule, which may be, for example, DNA or RNA, and are lengthened by a polymerase. They can also however act as probes.

### Probe

Probes are oligonucleotides or polynucleotides which hybridize on the target nucleic acid molecules. They are used for the direct or indirect detection of these target nucleic acid molecules. For this purpose, they can be coupled to fluorescent molecules or to molecules containing colouring agents. In addition they can be indirectly detected with an ELISA (enzyme-linked immunosorbent assay). In a special version they only produce a signal through FRET (Fluorescence Resonance Energy Transfer) when two probes hybridize adjacently in a defined manner. In this case a colouring agent on a probe is excited by a light beam and transfers its excitation energy to the colouring agent of the adjacent probe. This then emits light of a defined wavelength. They can also be used as primers.

### Multiplex PCR

A multiplex PCR is a Polymerase Chain Reaction or nucleic acid amplification reaction in which more than two primers are used which are not regarded as a forwards-backwards primer pair. With the presence of all nucleotide target molecules to be detected, this leads to the creation of at least two different amplicons. These amplicons should at least differ in the region in which the primers link, but they can also be allocated to completely different genes.

### Real-time Detection

In relation to this invention, real-time detection is defined as the simultaneous running of two processes: the detection of the DNA or RNA and a process which leads to the provision of a detectable amount of DNA or RNA. With this process the release of genomic DNA/RNA from cells may, for example, be involved or the enrichment of DNA/RNA from a complex mixture or the amplification of polynucleotides, *e.g*. through a PCR. Detection is the perception of a signal which correlates to the presence and possibly the amount of the DNA/RNA. In the case of the PCR this type of signal may increase with the increasing amplification of the target DNA. Real-time detection can be carried out also in a miniaturised form, *e.g*. on a chip. The signal can, for example, be produced through the fluorescent molecules of a probe, through radioactive molecules or through enzyme-coupled colour or fluorescence intensity.

The term real-time detection is synonymous to on-line detection.

### Derivatives of the Oligonucleotides According to the Invention

Derivatives or variants of the oligonucleotides according to the invention are taken to mean sequences which differ in at least one nucleotide from the specific sequences according to SEQ ID numbers 1-108, for example, by at least one base interchange, an insertion, deletion or addition. These also include oligonucleotides which are at least 80 % identical to one of the specific sequences according to SEQ ID numbers 1-108 and oligonucleotides with a comparable specificity of hybridization. The latter means that the derivative produces the same hybridization pattern with a specified sample containing nucleic acid, such as the oligonucleotide with one of the specific sequences with one of the SEQ ID numbers 1-108.

Throughout the application, variants of any nucleic acid molecule of any of SEQ ID NO 1-108 are contemplated as alternatives to the respective oligonucleotide of SEQ ID NO 1-108. A variant of a nucleic acid molecule of any of SEQ ID NO 1-108 is
a) a nucleic acid molecule which hybridizes under specific conditions with the respective nucleic acid molecule of any of SEQ ID NO 1-108;
b) a nucleic acid molecule which is at least 90% identical with the nucleic acid molecule of a); or
c) the complement of the nucleic acid molecule of SEQ ID NO 1-108 or of one of the variants a) to b).

Particular variants are also defined under "embodiments of the invention".

### Biochip

Biochip is taken to mean carriers for the high throughput of analyses as marketed, for example, by AFFYMETRIX. The chips enable the testing of numerous different nucleic acids on one carrier.

### Hybridization

The term "hybridization" is understood as the double strand formation of two identical or similar nucleic acid fragments (DNA, RNA, PNA, LNA). Specific hybridization is the term used if the hybridization is carried out under stringent conditions and gives a stable hybrid nucleic acid. In the terms of this invention, the feature "sequence which specifically hybridizes with a sequence according to (i)" refers to a sequence which hybridizes under stringent conditions with the sequence according to (i).

The stringency of the hybridization conditions depends on various factors such as the base composition of the hybrid, the level and geometry of mispairing between the two nucleic acids, as well as reaction parameters like ionic strength and the reaction temperature. Appropriate conditions can be determined based on thermodynamic calculations (SantaLucia et. al. 1996), which have been incorporated into a variety of computer programs known to those skilled in the art. Examples of stringent conditions are set forth in the example section below.

### Melting Curves and Melting Temperature

In relation to this invention, a melting curve analysis of a sample is defined as the following sequence of processes: the heating of the sample to a temperature high enough to denature the nucleic acids in the sample; the cooling of the sample to a temperature below the annealing temperature of the target nucleic acids; a slow heating of the sample with the simultaneous (real-time, online) detection of the dissociation (*i.e.,* melting) of the nucleic acids. Detection is the perception of a signal which correlates to the proportion of dissociated and not yet dissociated nucleic acids. In the case of a melting curve after a PCR with hybridization probes or with a double strand binding dye, this type of signal may decrease with the increasing temperature. An inflexion point of the signal course is defined as melting temperature. The melting temperature of a sample is the point at which half the probes or the double strand binding dye have melted off the target nucleic acids. For a better visualisation, the first negative derivative of the signal course can be plotted against the temperature; the melting temperatures then appear as peaks of the curve. A melting curve can also be carried out in a miniaturised form, e.g. on a chip.

With a preferred embodiment the nucleic acid to be examined is passed to a PCR. This has the result that specific amplicons are produced if nucleic acids of bacteria belonging to the baseline flora in environmental monitoring are present in the sample. Here in the simplest case, the PCR can be arranged as a simple linear PCR with only one oligonucleotide as primer, but preferably the PCR takes place however with so-called forwards and backwards primers for each genome section of the bacterial nucleic acid to be amplified.

With a further preferred embodiment various oligonucleotides and therefore various PCR runs are carried out in the form of a multiplex PCR. Here, different amplicons are created in the PCR in a single initiated reaction with the aid of the various oligonucleotides. Especially meaningful is the combination of oligonucleotides of this invention specific for the genera *Micrococcus, Kocuria, Kytococcus, Nesterenkonia,* and *Corynebacterium* with oligonucleotides for the detection of the genus *Staphylococcus.*

With a further preferred embodiment use is made of the so-called chip technology (biochips) in the detection method. Here, on one hand a large number of different analysis samples can be analysed on one chip in that the individual spots on the chip contain analysis material from different sources. On the other hand, the chip can carry a set of oligonucleotides, whereby each spot contains a specific oligonucleotide and this oligonucleotide pattern is brought into contact with analysis samples. In the case that the analysis material contains nucleic acids of bacteria belonging to the baseline flora in environmental monitoring, it hybridizes with the specific probes present on the chip and produces a corresponding signal pattern.

With a further preferred embodiment the detection method can include further steps, such as for example an amplification of the nucleic acid to be detected, whereby this preferably occurs using PCR and/or a southern hybridization with specific probes, whereby this hybridization occurs without prior amplification or after amplification of the nucleic acid to be detected is concluded. Furthermore, the nucleic acid to be detected can be detected using the ligase chain reaction. Finally, the nucleic acid to be detected can be enriched by isothermal nucleic acid amplification.

With a further preferred embodiment, the amplification of the target nucleic acid can also take place using real-time detection.

With a further preferred embodiment the amplification of the nucleic acid to be detected and/or the detection of the contained amplicons occurs on a biochip, whereby it is particularly preferable to carry out the amplification and detection on one chip.

According to the invention, as a means for carrying out the method described above, oligonucleotides are selected from a nucleic acid, comprising at least one sequence with one of the SEQ ID numbers 1-108 or derivatives thereof. The stated oligonucleotides can on one hand be used as primers within the scope of a PCR and on the other hand also as probes, for example within the scope of a Southern blot hybridization. Depending on the requirements of the desired detection, the specialist can form the suitable combination of oligonucleotides as primers or probes. Preferably, the stated oligonucleotides or combinations of them are used in the form of a kit for the classification of environmental organisms, whereby the kit also includes other reagents for the detection of bacteria or for conducting the detection reactions. In this respect, the reagents and enzymes required for the PCR and, where applicable, suitable carrier materials are also included, for example, such as is desired with the chip technology.

The oligonucleotides or oligonucleotide combinations according to the invention are therefore a suitable means for the specific identification of bacteria belonging to the baseline flora in environmental monitoring.

With the invention of the polymerase chain reaction it is possible to amplify individual DNA polynucleotides and then to detect them with extremely high sensitivity. This technology opens up substantial new opportunities, but also exhibits new problems. For example, incorrect fragments can easily be amplified, leading to incorrect positive results in the analysis. In addition, it is very difficult to select the diagnostic nucleic acid sequences characteristic to the target group of bacteria from the multitude of possibilities.

To avoid false-negative results, in a preferred embodiment of the invention, an amplification control nucleic acid is added during amplification. Accordingly, not only the microbial nucleic acid or a part thereof is amplified, but also the amplification control nucleic acid, which means that at least one amplification fragment in each case is created. The amplification control nucleic acid, *e.g*. a DNA fragment, is here an "internal standard molecule" which serves as an indicator for the effectiveness of the reaction (Ballagi-Pordany, Belak 1996). It is added in a defined quantity to the amplification reaction and amplified in parallel. The amplification control nucleic acid is preferably single or double stranded and may be of unlimited length. Amplification control nucleic acids with a length of up to a thousand nucleotides have proven successful.

### Description of the figures

Figure 1 shows the DNA region as well as the hybridization positions of the oligonucleotides of this invention.
Figure 2 shows the melting curve analysis in channel 705 for *Staphylococcus aureus* (solid line; positive amplification signal in channel 640, negative in channels 610 and 670) and *Micrococcus luteus* (dotted line; positive amplification signal in channel 670, negative in channels 610 and 640).
Figure 3 shows the melting curve analysis in channel 705 for some of the *Staphylococcus* isolates (positive amplification signal in channel 640, negative in channels 610 and 670).
Figure 4 shows the melting curve analysis in channel 705 for some of the isolates that showed a positive amplification signal in channel 670 and that were negative in channels 610 and 640.

### Examples

### Example 1

### Real-Time PCR for the Identification of Single Species of the genera Micrococcus and Kocuria

Genomic DNA of single colonies from agar plates was isolated using known standard methods. The DNA samples were then added to six PCR mixes with different primer pairs with the following composition (final volume 20 µl):

| **Component** | **Final Concentration** |
|---|---|
| H₂O | - |
| PCR buffer, 10 x conc. | 1 x conc. |
| MgCl₂ | 4 mM |
| dATP, dCTP, dGTP, dUTP | 200 µM each |
| Primer forward (see below) | 400 nM |
| Primer reverse (see below) | 400 nM |
| Taq DNA Polymerase | 0.125 U/µl |
| SYBR Green I | 0.25 x conc. |
| Sample DNA | var. |

| | **Mix 1** | **Mix 2** | **Mix 3** | **Mix 4** | **Mix 5** | **Mix 6** |
|---|---|---|---|---|---|---|
| Primer forward | SEQ ID No. 5 | SEQ ID No. 6 | SEQ ID No. 5 | SEQ ID No. 9 | SEQ ID No. 8 | SEQ ID No. 8 |
| Primer reverse | SEQ ID No. 64 | SEQ ID No. 64 | SEQ ID No. 76 | SEQ ID No. 81 | SEQ ID No. 87 | SEQ ID No. 83 |

The PCR was carried out on a LightCycle^{®} 1.1 instrument (Roche Diagnostics) using glass capillaries and the following time/temperature protocol:

| | | |
|---|---|---|
| Initial Denaturation | 95°C 2 min | |
| | | |
| Amplification (35 cycles) | 95°C 2 s | |
| | 62°C 20 s | |
| | 72°C 10 s | (fluorescence acquisition) |
| | | |
| Melting Curve | 95°C 0 s | |
| | 40°C 45 s | |
| | 95°C 0 s | (continuous fluorescence acquisition) |
| | | |
| Cooling | 40°C 30 s | |

The results are shown in table 3:

**Table 3: PCR results of Micrococcus and Kocuria isolates with different primer pairs.**

| **Species** | **Mix 1** | **Mix 2** | **Mix 3** | **Mix 4** | **Mix 5** | **Mix 6** |
|---|---|---|---|---|---|---|
| *Micrococcus luteus* | - | - | + | - | - | - |
| *Kocuria kristinae* | - | - | - | - | - | + |
| *Kocuria rosea* | - | - | - | + | - | - |
| *Kocuria varians* | + | - | - | - | - | - |
| *Kocuria camiphila* | - | + | - | - | - | - |
| *Kocuria palustris* | - | - | - | - | + | - |

| | | | | | | |
|---|---|---|---|---|---|---|
| + *= amplification curve in channel F1* / *- = no amplification curve in channel F1* | | | | | | |

Each of the primer combinations specifically detected only one of the tested species.

### Example 2

### Comparison of Conventional Identification Methods to Hybridization Probe-Based Real-Time PCR for the Genera Micrococcus, Kocuria, Kytococcus, and Staphylococcus

150 environmental isolates that have previously been identified by conventional methods (biochemical tests and microscopy) were collected from different pharmaceutical companies for testing with real-time PCR.

Genomic DNA of single colonies from agar plates was isolated using known standard methods. The DNA samples as well as an internal amplification control DNA were then added to the PCR mix with the following composition (final volume 20 µl):

| **Component** | **Final Concentration** |
|---|---|
| H₂O | - |
| PCR buffer, 10 x conc. | 1 x conc. |
| MgCl₂ | 4 mM |
| dATP, dCTP, dGTP, dUTP | 200 µM each |
| SEQ ID No. 5, 6, 8, 9 | 200 nM each |
| SEQ ID No. 7 | 100 nM |
| SEQ ID No. 64, 81, 86, 87, 92 | 250 nM each |
| SEQ ID No. 30 - 34 ^{a)} | 50 nM each |
| SEQ ID No. 13 ^{b)} | 250 nM |
| SEQ ID No. 55 ^{a)} | 100 nM |
| SEQ ID No. 58 ^{c)} | 100 nM |
| SEQ ID No. 93 - 101 | 400 nM each |
| SEQ ID No. 102 - 103 ^{a)} | 200 nM each |
| SEQ ID No. 104 - 105 ^{d)} | 200 nM each |
| SEQ ID No. 106 ^{a)} | 100 nM |
| SEQ ID No. 107 ^{c)} | 100 nM |
| SEQ ID No. 108 ^{c)} | 200 nM |
| Taq DNA Polymerase | 0.1 U/µl |
| Internal Amplification Control DNA | 10 fg/µl |
| Sample DNA | var. |

| | |
|---|---|
| a) 3'-labeled with Fluorescein b) 5'-labeled with Cy5 and 3'-blocked with a phosphate moiety c) 5'-labeled with Cy5.5 and 3'-blocked with a phosphate moiety d) 5'-labeled with LC-Red 640 and 3'-blocked with a phosphate moiety | |

The PCR was carried out on a LightCycle^{®} 2.0 instrument (Roche Diagnostics) using glass capillaries and the following time/temperature protocol:

| | | |
|---|---|---|
| Initial Denaturation | 95°C 2 min | |
| Amplification (35 cycles) | 95°C 2 s | |
| | 62°C 20 s | (fluorescence acquisition) |
| | 72°C 10 s | |
| | | |
| Melting Curve | 95°C 0 s | |
| | 40°C 45 s | |
| | 80°C 0 s | (continuous fluorescence acquisition) |
| | | |
| Cooling | 40°C 30 s | |

The software modules "Qualitative Detection" and "Tm Analysis" of the LightCycler^{®} 2.0 instrument software (Roche Diagnostics) were used to analyze amplification and melting curves, respectively.

Amplification of DNA of *Micrococcus* spp., *Kocuria* spp., *Kytococcus* spp., and *Nesterenkonia* spp. was detected in channel 670.

Amplification of DNA of *Staphylococcus* spp. was detected in channel 640.

Identification of *Micrococcus luteus* and *Staphylococcus aureus* was achieved through melting curve analysis in channel 705.

The internal amplification control was detected in channel 705 for all samples that were negative in channels 640 and 670. The suitability of these samples for PCR was shown with a consensus PCR system targeting the 16S rDNA of eubacteria (Lane 1991).

Conventional methods and PCR results corresponded in 113 of 150 cases (75.3 %). Deviating results were resolved by sequencing approx. 500 bp of the 16S rDNA for identification. The sequencing results confirmed the PCR in all cases. Thus, the superiority of the PCR method of this invention over conventional methods is clearly shown.

Table 4 shows a summary of the results.

**Table 4: Results of the comparative study, grouped by PCR results.**

| | **result conventional methods** | | |
|---|---|---|---|
| **PCR result, confirmed by sequencing** | **correct** | **incorrect** | **total** |
| negative - neither *Staphylococcus* nor *Micrococcus* / *Kocuria* / *Kytococcus* | 62 | 7 | 69 |
| positive for *Staphylococcus* | 15 | 7 | 22 |
| positive for *Micrococcus luteus* | 28 | 12 | 40 |
| positive for *Micrococcus* / *Kocuria* / *Kytococcus* (without *M*. *luteus)* | 8 | 11 | 19 |
| **Total** | **113** | **37** | **150** |

### Example 3

### Hybridization Probe-Based Real-Time PCR for the Genus Corynebacterium

Genomic DNA of single colonies from agar plates was isolated using known standard methods. The DNA samples were then added to the PCR mix with the following composition (final volume 20 µl):

| **Component** | **Final Concentration** |
|---|---|
| H₂O | - |
| PCR buffer, 10 x conc. | 1 x conc. |
| MgCl₂ | 4 mM |
| dATP, dCTP, dGTP, dUTP | 200 µM each |
| SEQ ID No. 1 | 750 nM |
| SEQ ID No. 90 | 400 nM |
| SEQ ID No. 47 - 49 ^{a)} | 100 nM each |
| SEQ ID No. 25 ^{b)} | 200 nM |
| Taq DNA Polymerase | 0.1 U/µl |
| Sample DNA | var. |

| | |
|---|---|
| *a) 3'-labeled with Fluorescein* *b) 5'-labeled with LC-Red 610 and 3'-blocked with a phosphate moiety* | |

The PCR was carried out on a LightCycle^{®} 2.0 instrument (Roche Diagnostics) using glass capillaries and the following time/temperature protocol:

| | | |
|---|---|---|
| Initial Denaturation | 95°C 2 min | |
| | | |
| Amplification (35 cycles) | 95°C 2 s | |
| | 62°C 20 s | (fluorescence acquisition) |
| | 72°C 10 s | |
| | | |
| Melting Curve | 95°C 0 s | |
| | 40°C 45 s | |
| | 80°C 0 s | (continuous fluorescence acquisition) |
| | | |
| Cooling | 40°C 30 s | |

Amplification of DNA of *Corynebacterium* spp. was detected in channel 610.

Table 5 lists the tested species and the respective PCR result.

**Table 5: Tested isolates and PCR results.**

| **Species** | **PCR Result** |
|---|---|
| *Actinomyces naeslundii* | - |
| *Brevibacterium casei* | - |
| *Brevibacterium sanguinis* | - |
| *Corynebacterium afermentans* | + |
| *Corynebacterium amycolatum* | + |
| *Corynebacterium aurimucosum* | + |
| *Corynebacterium coyleae* | + |
| *Corynebacterium genitalium* | + |
| *Corynebacterium glutamicum* | + |
| *Corynebacterium jeikeium* | + |
| *Corynebacterium kroppenstedtii* | + |
| *Corynebacterium macginleyi* | + |
| *Corynebacterium minutissimum* | + |
| *Corynebacterium mucifaciens* | + |
| *Corynebacterium simulans* | + |
| *Corynebacterium suicordis* | + |
| *Corynebacterium thomssenii* | + |
| *Corynebacterium tuberculostearicum* | + |
| *Exiguobacterium* sp. | - |
| *Gordonia polyisoprenivorans* | - |
| *Microbacterium aurum* | - |
| *Rothia dentocariosa* | - |
| *Williamsia serinedens* | - |

| | |
|---|---|
| + *= positive amplification signal in channel 610* / *- = no amplification signal in channel 610* | |

Only isolates of the genus *Corynebacterium* where detected, while even closely related organisms were PCR negative.

### Example 4

### Hybridization Probe-Based Real-Time PCR for the Most Common Bacterial Species in Environmental Monitoring (the Baseline Flora)

Genomic DNA of single colonies from agar plates was isolated using known standard methods. The DNA samples as well as an internal amplification control DNA were then added to the PCR mix with the following composition (final volume 20 µl):

| **Component** | **Final Concentration** |
|---|---|
| H₂O | - |
| PCR bluffer, 10 x conc. | 1 x conc. |
| MgCl₂ | 4mM |
| dATP, dCTP, dGTP, dUTP | 200 µM each |
| SEQ ID No: 1, 2, 7, 9 | 250 nM each |
| SEQ ID No: 67, 68, 69, 70, 71, 73, 75 | 600 nM each |
| SEQ ID No: 64, 79, 81, 86, 87 | 500 nM each |
| SEQ ID No: 10. 11, 55 ^{a)} | 150 nM each |
| SEQ ID No: 15 - 20 ^{b)} | 150 nM each |
| SEQ ID No: 36 - 40 ^{c)} | 225 nM each |
| SEQ ID No: 58 ^{d)} | 150 nM |
| SEQ ID No. 93 - 101 | 400 nM each |
| SEQ ID No. 102, 103 ^{a)} | 100 nM each |
| SEQ ID No. 104, 105 ^{e)} | 100 nM each |
| SEQ ID No. 106 ^{a)} | 100 nM |
| SEQ ID No. 107 ^{d)} | 100 nM |
| SEQ ID No. 108 ^{d)} | 200 nM |
| Taq DNA Polymerase | 0.1 U/µl |
| Internal Amplification Control DNA | 10 fg/µl |
| Sample DNA | var. |

| | |
|---|---|
| a) 3'-labeled with Fluorescein b) 5'-labeled with LC-Red 610 and 3'-blocked with a phosphate moiety c) 5'-labeled with Cy5 and 3'-blocked with a phosphate moiety d) 5'-labeled with Cy5.5 and 3'-blocked with a phosphate moiety e) 5'-labeled with LC-Red 640 and 3'-blocked with a phosphate moiety | |

The PCR was carried out on a LightCycler^{®} 2.0 instrument (Roche Diagnostics) using glass capillaries and the following time/temperature protocol:

| | | |
|---|---|---|
| Initial Denaturation | 95°C 2 min | |
| | | |
| Amplification (35 cycles) | 95°C 2 s | |
| | 62°C 20 s | (fluorescence acquisition) |
| | 72°C 10 s | |
| | | |
| Melting Curve | 95°C 0 s | |
| | 40°C 45 s | |
| | 80°C 0 s | (continuous fluorescence acquisition) |
| | | |
| Cooling | 40°C 30 s | |

The software modules "Qualitative Detection" and "Tm Analysis" of the LightCycle^{®} 2.0 instrument software (Roche Diagnostics) were used to analyze amplification and melting curves, respectively.

Amplification of DNA of *Corynebacterium* spp. was detected in channel 610, and a melting curve analysis was performed in channel 610.

Amplification of DNA of *Micrococcus* spp., *Kocuria* spp., *Kytococcus* spp., and *Nesterenkonia* spp. was detected in channel 670.

Amplification of DNA of *Staphylococcus* spp. and *Macrococcus* spp. was detected in channel 640, and differentiation of the two genera was performed via melting curve analysis in channel 640.

Identification of *Micrococcus luteus* and *Staphylococcus aureus* and additional classification was achieved through melting curve analysis in channel 705.

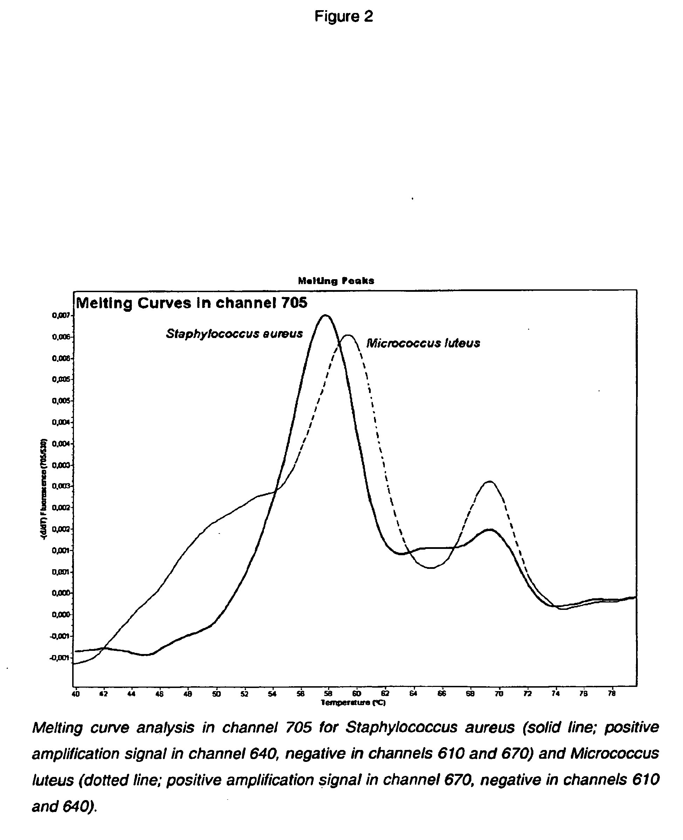

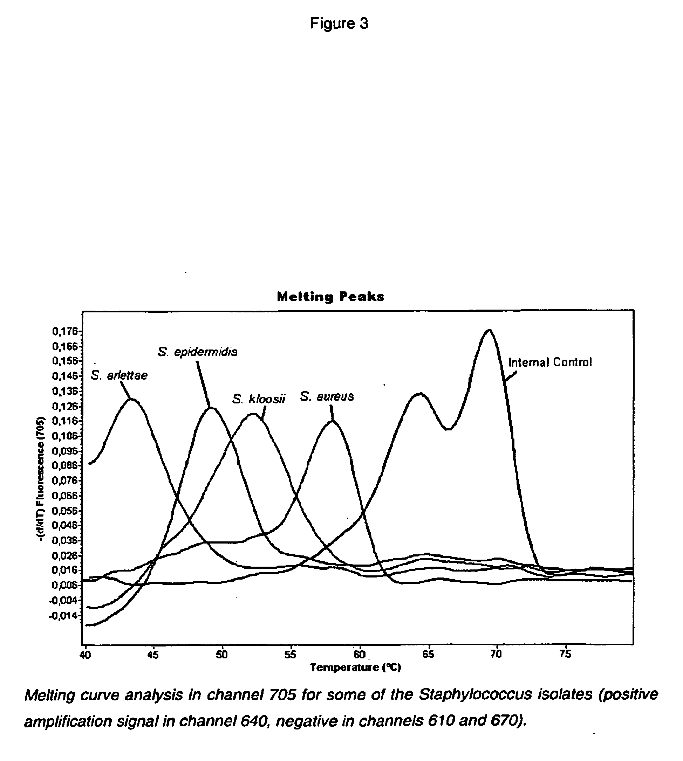

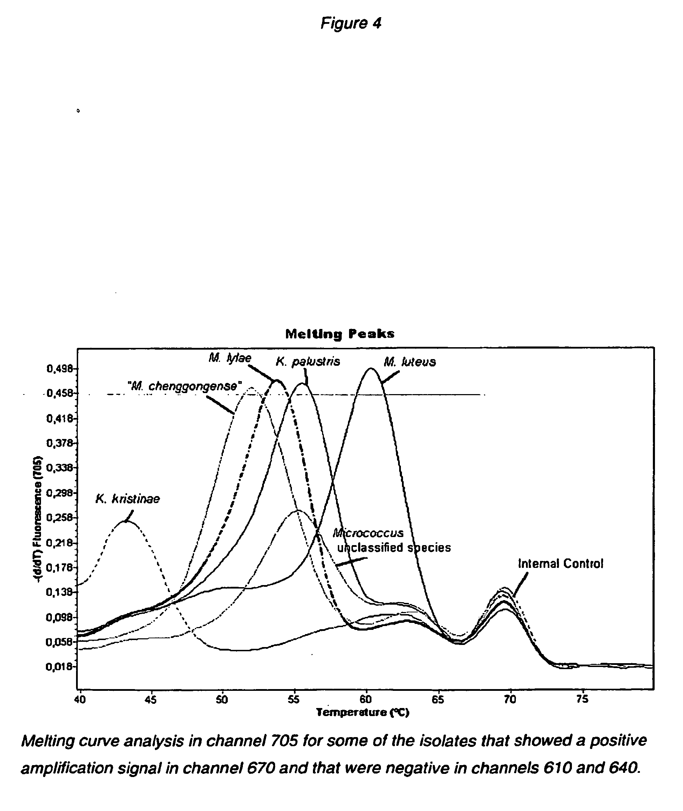

The results are shown in tables 6 - 10 and figures 2 - 4.

Phylogenetic classification of previously uncharacterized isolates was done by sequencing approx. 500 bp of the 16S rDNA. Isolates with less than 98 % sequence similarity to named species were denoted "sp." with their closest relative given in brackets.

All isolates that belonged to the genus *Corynebacterium* were detected in channel 610, no false negatives occurred. They all had a melting peak or shoulder at temperatures higher than 63°C in channel 610. Some coryneform bacteria had a melting curve in channel 610 as well, but with all peaks occurring at temperatures below 63°C.

All isolates that belonged to the genus *Staphylococcus* were detected in channel 640, no false negatives occurred. They all had a melting peak or shoulder at temperatures higher than 64°C in channel 640.

All isolates of *Staphylococcus aureus* could be identified by their specific melting peak temperature of approx. 58°C in channel 705. Additionally, the following species showed distinct melting peaks in channel 705:
- *Staphylococcus arlettae* at 43.4°C (one isolate tested)
- *Staphylococcus kloosii* at 52.1 °C (one isolate tested)

Furthermore, most isolates of the following species had a melting peak temperature between 48.8 and 49.3°C in channel 705:
- *Staphylococcus capitis* (7 of 7)
- *Staphylococcus caprae* (2 of 3)
- *Staphylococcus epidermidis* (16 of 16)
- *Staphylococcus haemolyticus* (11 of 16)
- *Staphylococcus hominis* (12 of 12; one isolate had a second peak)
- *Staphylococcus pasteuri* (1 of 1)
- *Staphylococcus saccharolyticus* (1 of 1)
- *Staphylococcus warneri* (7 of 7)

All isolates that belonged to the genus *Macrococcus* were detected in channel 640, no false negatives occurred. They could be distinguished from *Staphylococcus* spp. by melting curve analysis, as no peaks at temperatures higher than 64°C occurred in channel 640.

All isolates that belonged to the genera *Micrococcus, Kocuria, Kytococcus,* and *Nesterenkonia* were detected in channel 670, no false negatives occurred.

A broad spectrum of melting peak temperatures occurred in channel 705 for these genera, starting at approx. 41°C for *Kytococcus* spp. and the closely related *Nesterenkonia halobia.* Their melting peak range overlapped with that of the *Kocuria* species *K. carniphila, K. rhizophila,* and *K. varians* (41.0 - 42.2°C). *Kocuria kristinae* showed with 42.9 - 43.3°C slightly higher melting peak temperatures.

The second melting group comprised different *Micrococcus* spp. (but not *M. luteus*) and *Kocuria palustris*. The melting peak temperatures ranged from approx. 52°C for two isolates that phylogenetically belong to the new species *Micrococcus chenggongense* up to 56°C for *Kocuria palustris.*

All tested isolates of *Micrococcus luteus* could be identified by their specific melting peak temperature of approx. 60°C in channel 705.

*Kocuria rosea* (two isolates) and one *Kocuria* sp. similar to *K. rosea* did not have a specific melting curve in channel 705.

The internal amplification control was detected in channel 705 for all samples that were negative in channels 610, 640, and 670. The suitability of these samples for PCR was shown with a consensus PCR system targeting the 16S rDNA of eubacteria (Lane 1991).

**Table 6: Isolates of the genus Corynebacterium: positive amplification signal in channel 610, at least one melting peak or shoulder at temperatures above 63°C in melting curve channel 610.**

| **Species** | **Number of Isolates** |
|---|---|
| *Corynebacterium afermentans* | 9 |
| *Corynebacterium amycolatum* | 1 |
| *Corynebacterium appendicis* | 2 |
| *Corynebacterium aurimucosum* | 1 |
| *Corynebacterium caspium* | 1 |
| *Corynebacterium coyleae* | 4 |
| *Corynebacterium durum* | 1 |
| *Corynebacterium genitalium* | 1 |
| *Corynebacterium glutamicum* | 1 |
| *Corynebacterium imitans* | 1 |
| *Corynebacterium jeikeium* | 2 |
| *Corynebacterium kroppenstedtii* | 1 |
| *Corynebacterium kutscheri* | 1 |
| *Corynebacterium lipophiloflavum* | 1 |
| *Corynebacterium macginleyi* | 2 |
| *Corynebacterium minutissimum* | 3 |
| *Corynebacterium mucifaciens* | 5 |
| *Corynebacterium phocae* | 1 |
| *Corynebacterium riegelii* | 1 |
| *Corynebacterium simulans* | 1 |
| *Corynebacterium singulare* | 2 |
| *Corynebacterium suicordis* | 1 |
| *Corynebacterium thomssenii* | 1 |
| *Corynebacterium tuberculostearicum* | 22 |
| *Corynebacterium ureicelerivorans* | 4 |
| *Corynebacterium variabile* | 1 |
| *Corynebacterium xerosis* | 1 |
| *Corynebacterium* sp. (*C. urealyticum* < 96 %) | 1 |
| **Total: 28 different species of the genus *Corynebacterium*** | **73** |

**Table 7: Isolates of the genus Staphylococcus: positive amplification signal in channel 640, at least one melting peak or shoulder at temperatures 64°C in melting curve channel 640; characteristic melting peak temperatures in channel 705 are given in the last column of the table.**

| **Species** | **Number of Isolates** | **Tm 705 [°C]** |
|---|---|---|
| *Staphylococcus arlettae* | 1 | 43.4 |
| *Staphylococcus aureus* subsp. *anaerobius* | 1 | 57.8 |
| *Staphylococcus aureus* subsp. *aureus* | 47 | 57.2 - 58.4 |
| *Staphylococcus auricularis* | 1 | - |
| *Staphylococcus capitis* | 7 | 48.9 - 49.3 |
| *Staphylococcus caprae* | 3 | 48.8 - 49.1 (2 of 3) |
| *Staphylococcus camosus* | 1 | - |
| *Staphylococcus cohnii* | 3 | - |
| *Staphylococcus delphinii* | 1 | - |
| *Staphylococcus epidermidis* | 16 | 48.8 - 49.2 |
| *Staphylococcus equorum* | 5 | - |
| *Staphylococcus felis* | 1 | - |
| *Staphylococcus gallinarum* | 1 | - |
| *Staphylococcus haemolyticus* | 16 | 48.8 - 49.3 (11 of 16) |
| *Staphylococcus hominis* subsp. *horninis* | 6 | 49.0 - 49.2 |
| *Staphylococcus hominis* subsp. *novobiosepticus* | 6 | 49.0 - 49.3 |
| *Staphylococcus hyicus* | 1 | - |
| *Staphylococcus intermedius* | 1 | - |
| *Staphylococcus kloosii* | 1 | 52.1 |
| *Staphylococcus lentus* | 1 | - |
| *Staphylococcus lugdunensis* | 2 | - |
| *Staphylococcus muscae* | 1 | - |
| *Staphylococcus pasteuri* | 1 | 49.2 |
| *Staphylococcus pettenkoferi* | 1 | - |
| *Staphylococcus piscifermentans* | 1 | - |
| *Staphylococcus pseudointermedius* | 2 | - |
| *Staphylococcus saccharolyticus* | 1 | 48.9 |
| *Staphylococcus saprophyticus* | 3 | - |
| *Staphylococcus schleiferi* subsp. *coagulans* | 1 | - |
| *Staphylococcus schleiferi* subsp. *schleiferi* | 1 | - |
| *Staphylococcus sciuri* subsp. *sciuri* | 2 | - |
| *Staphylococcus simulans* | 1 | - |
| *Staphylococcus vitulinus* | 1 | - |
| *Staphylococcus warneri* | 7 | 48.9 - 49.2 |
| *Staphylococcus xylosus* | 2 | - |
| **Total: 32 species of the genus *Staphylococcus*** | **147** | **5 groups** |

**Table 8: Isolates of the genus Macrococcus: positive amplification signal in channel 640, all melting peaks at temperatures below 64°C in melting curve channel 640.**

| **Species** | **Number of Isolates** |
|---|---|
| *Macrococcus bovicus* | 1 |
| *Macrococcus carouselicus* | 1 |
| *Macrococcus caseolyticus* | 1 |
| *Macrococcus equipercicus* | 1 |
| **Total: 4 species of the genus *Macrococcus*** | **4** |

**Table 9: Isolates of the genera Micrococcus, Kocuria, Kytococcus, and Nesterenkonia: Positive amplification signal in channel 670; characteristic melting peak temperatures in channel 705 are given in the last column of the table.**

| **Species** | **Number of Isolates** | **Tm 705 [°C]** |
|---|---|---|
| *Kocuria carniphila* | 1 | 42.2 |
| *Kocuria kristinae* | 6 | 42.9 - 43.3 |
| *Kocuria palustris* | 5 | 55.1 - 55.7 |
| *Kocuria rhizophila* | 12 | 41.0 - 42.1 |
| *Kocuria rosea* | 2 | - |
| *Kocuria varians* | 1 | 42.1 |
| *Kocuria* sp. (*K. carniphila* < 97 %) | 1 | 42.0 |
| *Kocuria* sp. (*K. rosea* < 97 %) | 1 | - |
| *Kytococcus schroeteri* | 3 | 40.5 - 41.2 |
| *Kytococcus sedentarius* | 5 | 40.3 - 41.3 |
| *Micrococcus chenggongense* (unpublished) | 2 | 51.9 - 52.2 |
| *Micrococcus flavus* | 1 | 53.5 |
| *Micrococcus luteus* | 56 | 59.8 - 60.7 |
| *Micrococcus lylae* | 7 | 52.8 - 54.7 |
| *Micrococcus* sp. (*M. luteus* < 97 %) | 6 | 54.0 - 54.8 |
| *Nesterenkonia halobia* | 1 | 40.7 |
| **Total: 16 species of the 4 genera** | **110** | **4 groups** |

**Table 10: Isolates of non-target organisms: no amplification signal in channels 610, 640, and 670. Positive amplification signal in channel 705 (Internal Control).**

| **Species** | **Taxonomic Group** | **Number of Isolates** |
|---|---|---|
| *Achromobacter xylosoxidans* | *Betaproteobacteria* | 1 |
| *Actinomyces bovis* | *Actinomycetales* | 1 |
| *Actinomyces* sp. | *Actinomycetales* | 1 |
| *Aerococcus viridans* | *Lactobacillales* | 1 |
| *Agrococcus citreus* | *Actinomycetales* | 1 |
| *Arcobacter butzleri* | *Epsilonproteobacteria* | 1 |
| *Arthrobacter atrocyaneus* | *Actinomycetales* | 1 |
| *Arthrobacter crystallopoietes* | *Actinomycetales* | 1 |
| *Arthrobacter globiformis* | *Actinomycetales* | 1 |
| *Arthrobacter nicotianae* | *Actinomycetales* | 2 |
| *Arthrobacter oxydans* | *Actinomycetales* | 1 |
| *Arthrobacter pascens* | *Actinomycetales* | 1 |
| *Arthrobacter ramosus* | *Actinomycetales* | 2 |
| *Arthrobacter sulfonivorans* | *Actinomycetales* | 1 |
| *Bacillus cereus* | *Bacillales* | 3 |
| *Bacillus licheniformis* | *Bacillales* | 2 |
| *Bacillus pumilus* | *Bacillales* | 2 |
| *Bacillus subtilis* | *Baciliales* | 1 |
| *Brachybacterium* sp. (*B. faecium 97* %) | *Actinomycetales* | 1 |
| *Brevibacillus agri* | *Bacillales* | 1 |
| *Brevibacterium casei* | *Actinomycetales* | 8 |
| *Brevibacterium celere* | *Actinomycetales* | 3 |
| *Brevibacterium epidermidis* | *Actinomycetales* | 2 |
| *Brevibacterium linens* | *Actinomycetales* | 1 |
| *Brochothrix campestris* | *Baciliales* | 1 |
| *Buttiauxella agrestis* | *Gammaproteobacteria* | 1 |
| *Campylobacter curvus* | *Epsilonproteobacteria* | 1 |
| *Carnobacterium maltaromaticum* | *Lactobacillales* | 2 |
| *Citrobacter freundii* | *Gammaproteobacteria* | 1 |
| *Dermacoccus nishinomiyaensis* | *Actinomycetales* | 5 |
| *Desulfovibrio intestinalis* | *Deltaproteobacteria* | 1 |
| *Dietzia cinnamea* | *Actinomycetales* | 2** |
| *Enterobacter aerogenes* | *Gammaproteobacteria* | 1 |
| *Enterobacter cloacae* | *Gammaproteobacteria* | 1 |
| *Enterobacter faecalis* | *Gammaproteobacteria* | 1 |
| *Enterobacter intermedius* | *Gammaproteobacteria* | 1 |
| *Enterococcus casseliflavus* | *Lactobacillales* | 1 |
| *Escherichia coli* | *Gammaproteobacteria* | 1 |
| *Exiguobacterium aurantiacum* | *Bacillales* | 1 |
| *Gemella morbillorum* | *Bacillales* | 1 |
| *Geobacillus stearothermophilus* | *Bacillales* | 1 |
| *Gordonia polyisoprenivorans* | *Actinomycetales* | 1** |
| *Lactobacillus brevis* | *Lactobacillales* | 1 |
| *Lactobacillus parabuchneri* | *Lactobacillales* | 1 |
| *Lactococcus lactis* | *Lactobacillales* | 1 |
| *Listeria grayi* | *Bacillales* | 1 |
| *Listeria ivanovii* | *Bacillales* | 1 |
| *Listeria monocytogenes* | *Bacillales* | 2 |
| *Listeria seeligeri* | *Bacillales* | 1 |
| *Listeria welshimeri* | *Bacillales* | 1 |
| *Microbacterium aoyamense* | *Actinomycetales* | 1 |
| *Microbacterium aurum* | *Actinomycetales* | 1 |
| *Microbacterium flavescens* | *Actinomycetales* | 1 |
| *Microbacterium hominis* | *Actinomycetales* | 1 |
| *Microbacterium lacticum* | *Actinomycetales* | 1 |
| *Microbacterium testaceum* | *Actinomycetales* | 1 |
| *Morganella morganii* | *Gammaproteobacteria* | 1 |
| *Nocardioides simplex* | *Actinomycetales* | 1** |
| *Nocardiopsis dassonvillei* | *Actinomycetales* | 1 |
| *Paenibacillus thailandensis* | *Bacillales* | 1 |
| *Propionibacterium avidum* | *Actinomycetales* | 1 |
| *Proteus mirabilis* | *Gammaproteobacteria* | 1 |
| *Providencia stuartii* | *Gammaproteobacteria* | 1 |
| *Pseudomonas aeruginosa* | *Gammaproteobacteria* | 1 |
| *Pseudomonas psychrotolerans* | *Gammaproteobacteria* | 1 |
| *Psychrobacter cibarius* | *Gammaproteobacteria* | 1 |
| *Psychrobacter fozii* | *Gammaproteobacteria* | 2 |
| *Psychrobacter maritimus* | *Gammaproteobacteria* | 4 |
| *Psychrobacter okhotskensis* | *Gammaproteobacteria* | 1 |
| *Ralstonia pickettii* | *Betaproteobacteria* | 1 |
| *Rathayibacter tritici* | *Actinomycetales* | 1 |
| *Rhodococcus erythropolis* | *Actinomycetales* | 3** |
| *Rhodococcus opacus* | *Actinomycetales* | 1** |
| *Rhodococcus rhodochrous* | *Actinomycetales* | 1** |
| *Rothia amarae* | *Actinomycetales* | 1 |
| *Rothia dentocariosa* | *Actinomycetales* | 2 |
| *Rothia mucilaginosa* | *Actinomycetales* | 1 |
| *Saccharomyces cerevisiae* | *Ascomycota* | 1 |
| *Salinicoccus roseus* | *Bacillales* | 1 |
| *Salmonella* Enteritidis | *Gammaproteobacteria* | 1 |
| *Salmonella* Typhimurium | *Gammaproteobacteria* | 1 |
| *Serratia ficaria* | *Gammaproteobacteria* | 1 |
| *Serratia plymuthica* | *Gammaproteobacteria* | 1 |
| *Shigella boydii* | *Gammaproteobacteria* | 1 |
| *Shigella sonnei* | *Gammaproteobacteria* | 1 |
| *Stenotrophomonas maltophilia* | *Gammaproteobacteria* | 1 |
| *Streptococcus gallolyticus* subsp. *macedonicus* | *Lactobacillales* | 1 |
| *Williamsia serinedens* | *Actinomycetales* | 1** |
| *Yarrowia lipolytica* | *Ascomycota* | 1 |
| unclassified *Bacillales* bacterium (*Jeotgalicoccus halotolerans* < 95 %) | *Bacillales* | 2 |
| unclassified *Propionibacteriaceae* bacterium | *Actinomycetales* | 2 |
| (*Microlunatus ginsengioli* < 95 %) | | |
| **Total: 89 Gram-positive bacteria (58 *Actinomycetales,* 23 *Bacillales,* 8 *Lactobacillales*) / 32 Gram-negative bacteria (2 *Beta*-, *27 Gamma*-, 1 *Delta*-, 2 *Epsilonproteobacteria*) / 2 yeasts** | | **123** |

| | | |
|---|---|---|
| ***These coryneform bacteria showed a melting curve in channel 610 with all melting peaks at temperatures below 63°C.* | | |

### References

Ballagi-Pordany, A.; Belak, S. (1996): The use of mimics as internal standards to avoid false negatives in diagnostic PCR. Mol Cell Probes, 10 (3), 159-164.
Bergey, D H (2001): Bergey's Manual of Systematic Bacteriology. 2. Ed., New York: Springer.
Brigante, Gioconda; Menozzi, Maria Grazia; Pini, Beatrice; Porta, Rosaria; Somenzi, Pietro; Sciacca, Agata et al. (2008): Identification of Coagulase-Negative Staphylococci by Using the BD Phoenix System in the Low-Inoculum Mode. J. Clin. Microbiol., 46 (11), 3826-3828.
Delmas, Julien; Chacornac, Jean Paul; Robin, Frederic; Giammarinaro, Philippe; Talon, Regine; Bonnet, Richard (2008): Evaluation of the Vitek 2 System with a Variety of Staphylococcus Species. J. Clin. Microbiol., 46 (1), 311-313.
Hitschfeld, Robert (2005): Computergestütztes Umgebungsmonitoring in der pharmazeutischen Parenteralia- Produktion. Dissertation. Berlin. Freie Universität Berlin, Fachbereich Biologie, Chemie, Pharmazie.
Lane, D. J. (1991): 16S/23S rRNA sequencing. In: Stackebrandt, Erko; Goodfellow, Michael (ed.): Nucleic acid techniques in bacterial systematics. Chichester, UK: John Wiley & Sons Ltd., 115-175.
Merker, P.; Grohmann, L.; Petersen, R.; Ladewig, J.; Gerbling, K. P.; Lauter, F. R. (2000): Alternative microbial testing: a novel DNA-based detection system for specified microorganisms in pharmaceutical preparations. PDA J Pharm Sci Technol, 54 (6), 470-477.
SantaLucia, J. Jr.; Allawi, H. T.; Seneviratne, P. A. (1996): Improved Nearest-Neighbor Parameters for Predicting DNA Duplex Stability. Biochem., 35 (11), 3555 - 3562.

## Claims

1. A kit comprising a combination of nucleic acid molecules suitable in the detection of the taxonomic unit comprising or consisting of the genera *Micrococcus, Kocuria, Kytococcus, Nesterenkonia,* the combination comprising
(i)
(a) the combination of SEQ ID NOs: 2, 7 and 9;
(b) the combination of a nucleic acid molecule which hybridizes under stringent conditions with SEQ ID NO: 2, a nucleic acid molecule which hybridizes under stringent conditions with SEQ ID NO: 7 and a nucleic acid molecule which hybridizes under stringent conditions with SEQ ID NO: 9;
(c) the combination of a nucleic acid molecule which is at least 90 % identical with SEQ ID NO: 2, a nucleic acid molecule which is at least 90 % identical with SEQ ID NO:7, a nucleic acid molecule which is at least 90 % identical with SEQ ID NO: 9;or
(d) the combination of the complements of the nucleic acid molecules of (i) ((a), (b) or (c); together with
(ii)
(a) the combination of SEQ ID NOs: 64, 79, 81, 86, and 87;
(b) the combination of a nucleic acid molecule which hybridizes under stringent conditions with SEQ ID NO: 64, a nucleic acid molecule which hybridizes under stringent conditions with SEQ ID NO: 79, a nucleic acid molecule which hybridizes under stringent conditions with SEQ ID NO: 81, a nucleic acid molecule which hybridizes under stringent conditions with SEQ ID NO: 86, and a nucleic acid molecule which hybridizes under stringent conditions with SEQ ID NO: 87;
(c) the combination of a nucleic acid molecule which is at least 90 % identical with SEQ ID NO: 64, a nucleic acid molecule which is at least 90 % identical with SEQ ID NO:79, a nucleic acid molecule which is at least 90 % identical with SEQ ID NO: 81, a nucleic acid molecule which is at least 90 % identical with SEQ ID NO:86, and a nucleic acid molecule which is at least 90 % identical with SEQ ID NO: 87; or
(d) the combination of the complements of the nucleic acid molecules of (ii) (a), (b) or (c)

2. The kit of claim 1, further comprising a nucleic acid selected from
(a) SEQ ID NOs: 10, 11, 12, 36, 37, 38, 39 or 40;
(b) a nucleic acid molecule which hybridizes under stringent conditions with a nucleic acid molecule of (a);
(c) a nucleic acid molecule which is at least 90 % identical with the nucleic acid molecule of (a); or
(d) the complement of a nucleic acid molecule of (a) to (c).

3. The kit of any of claims 1 to 2, which is also suitable for use in the detection of the genus *Corynebacterium,* the combination further comprising
i) SEQ ID NO: 1; or a variant thereof; and
one of SEQ ID NOs: 67, 68, 69, 70, 71, 73, 75; or a respective variant of one of these; or
ii) SEQ ID NOs: 3 and 4 or a respective variant of one of these; and
one of the SEQ ID NOs: 46, 90 or 91; or a respective variant of one of these,
wherein a variant of a nucleic acid molecule of i) or ii) is
a) a nucleic acid molecule which hybridizes under stringent conditions with the nucleic acid molecule;
b) a nucleic acid molecule which is at least 90 % identical with the nucleic acid molecule of a); or
c) the complement of the nucleic acid molecule or of one of the variants a) to b).

4. The kit of any of claims 1 to 4, which is also suitable for use in the detection of the genus *Staphylococcus,* the combination further comprising
i) SEQ ID NO: 93; or a variant thereof; and
one of SEQ ID NOs: 94-101; or a respective variant of one of these;
wherein a variant of a nucleic acid molecule of i) is
a) a nucleic acid molecule which hybridizes under stringent conditions with the nucleic acid molecule;
b) a nucleic acid molecule which is at least 90 % identical with the nucleic acid molecule of a); or
c) the complement of the nucleic acid molecule or of one of the variants a) to b).

5. A method for amplifying or identifying bacterial DNA of the taxonomic unit comprising or consisting of the genera *Micrococcus, Kocuria, Kytococcus, Nesterenkonia* in a sample, in which
a) in a first amplification step the DNA of the taxonomic unit is amplified with conserved primers, wherein the primers used in the first amplification step comprise all of the nucleic acids as defined in claim 1 (i) (a) or all of the nucleic acids as defined in claim 1 (i) (b) or all of the nucleic acids as defined in claim 1 (i) (c); together with all of the nucleic acids as defined in claim 1 (ii) (a) or all of the nucleic acids as defined in claim 1 (ii) (b) or all of the nucleic acids as defined in claim 1 (ii) (c).

6. The method of claim 5, wherein
b) in a further detection step, the DNA fragments obtained by amplification step a) are detected by means of probes,
wherein the probes used in step b) comprise one or more of the nucleic acids as defined in claim 2 (a) to (d).

7. The method of any of claims 5 or 6, which in the same sample also allows for amplifying or identifying the genus *Corynebacterium* and/or *Staphylococcus,* in which method
i) in a second amplification step, which is simultaneous or subsequent to the first amplification step, the DNA of the genus *Corynebacterium* is amplified with conserved primers, and/or
ii) in a third amplification step, which is simultaneous or subsequent to the first amplification step, the DNA of the genus *Staphylococcus* is amplified with conserved primers.

8. The method of any of claims 5 to 7, wherein the primers used in the second amplification step comprise one or more of the nucleic acids selected from
a) SEQ ID NOs: 1, 3 or 4,
b) SEQ ID NOs: 46, 90, 91, 67, 68, 69, 70, 71, 73, 75, 72 or 74;
c) a nucleic acid molecule which hybridizes under stringent conditions with the nucleic acid molecule of any of a) to b);
d) a nucleic acid molecule which is at least 90 % identical with the nucleic acid molecule of a), or b); or
e) the complement of the nucleic acid molecule of any of a) or b).

9. The method of any of claims 5 to 8, wherein the primers used in the third amplification step comprise one or more of the nucleic acids selected from
a) SEQ ID NO: 93;
b) SEQ ID NOs: 94, 95, 96, 97, 98, 99, 100 or 101;
c) a nucleic acid molecule which hybridizes under stringent conditions with the nucleic acid molecule of any of a) to b);
d) a nucleic acid molecule which is at least 90 % identical with the nucleic acid molecule of a), or b); or
e) the complement of the nucleic acid molecule of any of a) or b).

10. The method of any of claims 5 to 9, further allowing the amplification or detection of one or more of the genera *Micrococcus, Kocuria, Kytococcus* and/or *Nesterenkonia* or one or more species within one or more of these genera.

11. The method of any of claims 5 to 10, further allowing the amplification or detection of one or more of *Micrococcus luteus, Staphylococcus aureus* or *Dermacoccus nishinomiyaensis.*

## Patentansprüche

1. Kit, umfassend eine Kombination von Nucleinsäuremolekülen, die beim Nachweis der taxonomischen Einheit, umfassend die Gattungen *Micrococcus, Kocuria, Kytococcus, Nesterenkonia* oder daraus bestehend, geeignet sind, wobei die Kombination umfasst
(i)
(a) die Kombination der SEQ ID NO: 2, 7 und 9;
(b) die Kombination eines Nucleinsäuremoleküls, das unter stringenten Bedingungen mit SEQ ID NO: 2 hybridisiert, eines Nucleinsäuremoleküls, das unter stringenten Bedingungen mit SEQ ID NO: 7 hybridisiert, und eines Nucleinsäuremoleküls, das unter stringenten Bedingungen mit SEQ ID NO: 9 hybridisiert;
(c) die Kombination eines Nucleinsäuremoleküls, das zu mindestens 90% mit SEQ ID NO: 2 identisch ist, eines Nucleinsäuremoleküls, das zu mindestens 90% mit SEQ ID NO: 7 identisch ist, eines Nucleinsäuremoleküls, das zu mindestens 90% mit SEQ ID NO: 9 identisch ist, oder
(d) die Kombination der Komplemente der Nucleinsäuremoleküle nach (i) (a), (b) oder (c); zusammen mit
(ii)
(a) der Kombination der SEQ ID NO: 64, 79, 81, 86 und 87;
(b) der Kombination eines Nucleinsäuremoleküls, das unter stringenten Bedingungen mit SEQ ID NO: 64 hybridisiert, eines Nucleinsäuremoleküls, das unter stringenten Bedingungen mit SEQ ID NO: 79 hybridisiert, eines Nucleinsäuremoleküls, das unter stringenten Bedingungen mit SEQ ID NO: 81 hybridisiert, eines Nucleinsäuremoleküls, das unter stringenten Bedingungen mit SEQ ID NO: 86 hybridisiert, und eines Nucleinsäuremoleküls, das unter stringenten Bedingungen mit SEQ ID NO: 87 hybridisiert;
(c) der Kombination eines Nucleinsäuremoleküls, das zu mindestens 90% mit SEQ ID NO: 64 identisch ist, eines Nucleinsäuremoleküls, das zu mindestens 90% mit SEQ ID NO: 79 identisch ist, eines Nucleinsäuremoleküls, das zu mindestens 90% mit SEQ ID NO: 81 identisch ist, eines Nucleinsäuremoleküls, das zu mindestens 90% mit SEQ ID NO: 86 identisch ist, und eines Nucleinsäuremoleküls, das zu mindestens 90% mit SEQ ID NO: 87 identisch ist, oder
(d) der Kombination der Komplemente der Nucleinsäuremoleküle nach (ii) (a), (b) oder (c).

2. Kit nach Anspruch 1, ferner umfassend eine Nucleinsäure, ausgewählt aus
(a) SEQ ID NO: 10, 11, 12, 36, 37, 38, 39 oder 40;
(b) einem Nucleinsäuremolekül, das unter stringenten Bedingungen mit einem Nucleinsäuremolekül nach (a) hybridisiert;
(c) einem Nucleinsäuremolekül, das zu mindestens 90% mit dem Nucleinsäuremolekül nach (a) identisch ist, oder
(d) dem Komplement eines Nucleinsäuremoleküls nach (a) bis (c).

3. Kit nach einem der Ansprüche 1 bis 2, der auch zur Verwendung beim Nachweis der Gattung *Corynebacterium* geeignet ist, wobei die Kombination ferner umfasst
i) SEQ ID NO: 1 oder eine Variante davon und
eine aus SEQ ID NO: 67, 68, 69, 70, 71, 73, 75 oder eine jeweilige Variante von einer von diesen oder
ii) SEQ ID NO: 3 und 4 oder eine jeweilige Variante von einer von diesen und eine aus den SEQ ID NO: 46, 90 oder 91 oder eine jeweilige Variante von einer von diesen,
wobei es sich bei einer Variante eines Nucleinsäuremoleküls nach i) oder ii)
a) um ein Nucleinsäuremolekül handelt, das unter stringenten Bedingungen mit dem Nucleinsäuremolekül hybridisiert;
b) um ein Nucleinsäuremolekül handelt, das zu mindestens 90% mit dem Nucleinsäuremolekül nach a) identisch ist, oder
c) um das Komplement des Nucleinsäuremoleküls oder einer der Varianten a) bis b) handelt.

4. Kit nach einem der Ansprüche 1 bis 4, der auch zur Verwendung beim Nachweis der Gattung *Staphylococcus* geeignet ist, wobei die Kombination ferner umfasst
(i) SEQ ID NO: 93 oder eine Variante davon und
eine aus SEQ ID NO: 94 - 101 oder eine jeweilige Variante von einer von diesen;
wobei es sich bei einer Variante eines Nucleinsäuremoleküls nach i)
a) um ein Nucleinsäuremolekül handelt, das unter stringenten Bedingungen mit dem Nucleinsäuremolekül hybridisiert;
b) um ein Nucleinsäuremolekül handelt, das zu mindestens 90% mit dem Nucleinsäuremolekül nach a) identisch ist, oder
c) um das Komplement des Nucleinsäuremoleküls oder einer der Varianten a) bis b) handelt.

5. Verfahren zum Amplifizieren oder Identifizieren von Bakterien-DNA der taxonomischen Einheit, umfassend die Gattungen *Micrococcus, Kocuria, Kytococcus, Nesterenkonia* oder daraus bestehend, in einer Probe, wobei
(a) in einem ersten Amplifikationsschritt die DNA der taxonomischen Einheit mit konservierten Primern amplifiziert wird, wobei die in dem ersten Amplifikationsschritt verwendeten Primer alle in Anspruch 1 (i) (a) definierten Nucleinsäuren oder alle in Anspruch 1 (i) (b) definierten Nucleinsäuren oder alle in Anspruch 1 (i) (c) definierten Nucleinsäuren zusammen mit allen in Anspruch 1 (ii) (a) definierten Nucleinsäuren oder allen in Anspruch 1 (ii) (b) definierten Nucleinsäuren oder allen in Anspruch 1 (ii) (c) definierten Nucleinsäuren umfassen.

6. Verfahren nach Anspruch 5, wobei
(b) in einem weiteren Nachweisschritt die durch den Amplifikationsschritt (a) erhaltenen DNA-Fragmente mittels Sonden nachgewiesen werden,
wobei die in Schritt b) verwendeten Sonden eine oder mehrere der in Anspruch 2 (a) bis (d) definierten Nucleinsäuren umfassen.

7. Verfahren nach einem der Ansprüche 5 oder 6, das in der gleichen Probe auch das Amplifizieren oder Identifizieren der Gattung *Corynebacterium* und/oder *Staphylococcus* ermöglicht, wobei bei dem Verfahren
i) in einem zweiten Amplifikationsschritt, der gleichzeitig mit dem ersten Amplifikationsschritt oder anschließend stattfindet, die DNA der Gattung *Corynebacterium* mit konservierten Primern amplifiziert wird, und/oder
ii) in einem dritten Amplifikationsschritt, der gleichzeitig mit dem ersten Amplifikationsschritt oder anschließend stattfindet, die DNA der Gattung *Staphylococcus* mit konservierten Primern amplifiziert wird.

8. Verfahren nach einem der Ansprüche 5 bis 7, wobei die in dem zweiten Amplifikationsschritt verwendeten Primer eine oder mehrere der Nucleinsäuren umfassen, ausgewählt aus
a) SEQ ID NO: 1, 3 oder 4,
b) SEQ ID NO: 46, 90, 91, 67, 68, 69, 70, 71, 73, 75, 72 oder 74;
c) einem Nucleinsäuremolekül, das unter stringenten Bedingungen mit dem Nucleinsäuremolekül nach einem von a) bis b) hybridisiert;
d) einem Nucleinsäuremolekül, das zu mindestens 90% mit dem Nucleinsäuremolekül nach a) oder b) identisch ist, oder
e) dem Komplement des Nucleinsäuremoleküls nach einem von a) oder b).

9. Verfahren nach einem der Ansprüche 5 bis 8, wobei die in dem dritten Amplifikationsschritt verwendeten Primer eine oder mehrere der Nucleinsäuren umfassen, ausgewählt aus
a) SEQ ID NO: 93;
b) SEQ ID NO: 94, 95, 96, 97, 98, 99, 100 oder 101;
c) einem Nucleinsäuremolekül, das unter stringenten Bedingungen mit dem Nucleinsäuremolekül nach einem von a) bis b) hybridisiert;
d) einem Nucleinsäuremolekül, das zu mindestens 90% mit dem Nucleinsäuremolekül nach a) oder b) identisch ist, oder
e) dem Komplement des Nucleinsäuremoleküls nach einem von a) oder b).

10. Verfahren nach einem der Ansprüche 5 bis 9, das ferner die Amplifikation oder den Nachweis von einer oder mehreren der Gattungen *Micrococcus, Kocuria, Kytococcus* und/oder *Nesterenkonia* oder von einer oder mehreren Spezies innerhalb einer oder mehrerer dieser Gattungen ermöglicht.

11. Verfahren nach einem der Ansprüche 5 bis 10, das ferner die Amplifikation oder den Nachweis von einer oder mehreren aus *Micrococcus luteus, Staphylococcus aureus* oder *Dermacoccus nishinomiyaensis* ermöglicht.

## Revendications

1. Kit comprenant une combinaison de molécules d'acide nucléique adapté pour la détection de l'unité taxonomique constituée par ou comprenant les genres *Micrococcus, Kocuria, Kytococcus* et *Nesterenkonia,* la combinaison comprenant
(i)
(a) la combinaison des SEQ ID n° 2, 7 et 9 ;
(b) la combinaison d'une molécule d'acide nucléique qui s'hybride sous des conditions rigoureuses avec la SEQ ID n° 2, d'une molécule d'acide nucléique qui s'hybride sous des conditions rigoureuses avec la SEQ ID n° 7, et d'une molécule d'acide nucléique qui s'hybride sous des conditions rigoureuses avec la SEQ ID n° 9 ;
(c) la combinaison d'une molécule d'acide nucléique qui est au moins 90 % identique à la SEQ ID n° 2, d'une molécule d'acide nucléique qui est au moins 90 % identique à la SEQ ID n° 7, et d'une molécule d'acide nucléique qui est au moins 90 % identique à la SEQ ID n° 9 ; ou
(d) la combinaison des complémentaires des molécules d'acide nucléique de (i) (a), (b) ou (c) ; conjointement à
(ii)
(a) la combinaison des SEQ ID n° 64, 79, 81, 86 et 87 ;
(b) la combinaison d'une molécule d'acide nucléique qui s'hybride sous des conditions rigoureuses avec la SEQ ID n° 64, d'une molécule d'acide nucléique qui s'hybride sous des conditions rigoureuses avec la SEQ ID n° 79, d'une molécule d'acide nucléique qui s'hybride sous des conditions rigoureuses avec la SEQ ID n° 81, d'une molécule d'acide nucléique qui s'hybride sous des conditions rigoureuses avec la SEQ ID n° 86, et d'une molécule d'acide nucléique qui s'hybride sous des conditions rigoureuses avec la SEQ ID n° 87 ;
(c) la combinaison d'une molécule d'acide nucléique qui est au moins 90 % identique à la SEQ ID n° 64, d'une molécule d'acide nucléique qui est au moins 90 % identique à la SEQ ID n° 79, d'une molécule d'acide nucléique qui est au moins 90 % identique à la SEQ ID n° 81, d'une molécule d'acide nucléique qui est au moins 90 % identique à la SEQ ID n° 86, et d'une molécule d'acide nucléique qui est au moins 90 % identique à la SEQ ID n° 87 ; ou
(d) la combinaison des complémentaires des molécules d'acide nucléique du point (ii) (a), (b) ou (c).

2. Kit selon la revendication 1, comprenant en outre un acide nucléique sélectionné parmi
(a) les SEQ ID n° 10, 11, 12, 36, 37, 38, 39 ou 40 ;
(b) une molécule d'acide nucléique qui s'hybride sous des conditions rigoureuses avec une molécule d'acide nucléique de (a) ;
(c) une molécule d'acide nucléique qui est au moins 90 % identique à la molécule d'acide nucléique du point (a) ; ou
(d) le complémentaire d'une molécule d'acide nucléique des points (a) à (c).

3. Kit selon l'une quelconque des revendications 1 et 2, qui est également adapté pour être utilisé pour la détection du genre *Corynebacterium,* la combinaison comprenant en outre
i) la SEQ ID n° 1 ; ou une variante de celle-ci ; et
une des SEQ ID n° 67, 68, 69, 70, 71, 73, 75 ; ou une variante respective d'une de ces séquences ; ou
ii) les SEQ ID n° 3 et 4 ; ou une variante respective d'une de ces séquences ; et
une des SEQ ID n° 46, 90 ou 91 ; ou une variante respective d'une de ces séquences,
dans lequel une variante d'une molécule d'acide nucléique du point i) ou ii) est
a) une molécule d'acide nucléique qui s'hybride sous des conditions rigoureuses avec la molécule d'acide nucléique ;
b) une molécule d'acide nucléique qui est au moins 90 % identique à la molécule d'acide nucléique du point a) ; ou
c) le complémentaire de la molécule d'acide nucléique ou d'une des variantes a) et b).

4. Kit selon l'une quelconque des revendications 1 à 4, qui est également adapté pour être utilisé dans la détection du genre *Staphylococcus,* la combinaison comprenant en outre
i) la SEQ ID n° 93 ; ou une variante de celle-ci ; et
l'une des séquences SEQ ID n° 94-101 ; ou une variante respective d'une de ces séquences ;
dans lequel une variante d'une molécule d'acide nucléique du point i) est
a) une molécule d'acide nucléique qui s'hybride sous des conditions rigoureuses avec la molécule d'acide nucléique ;
b) une molécule d'acide nucléique qui est au moins 90 % identique à la molécule d'acide nucléique du point a) ; ou
c) le complémentaire de la molécule d'acide nucléique ou d'une des variantes a) et b).

5. Procédé d'amplification ou d'identification d'ADN bactérien de l'unité taxonomique constituée par ou comprenant les genres *Micrococcus, Kocuria, Kytococcus* et *Nesterenkonia* dans un échantillon, dans lequel
a) dans une première étape d'amplification, l'ADN de l'unité taxonomique est amplifié avec des amorces conservées, dans lequel les amorces utilisées à la première étape d'amplification comprennent tous les acides nucléiques définis dans la revendication 1 (i) (a), ou tous les acides nucléiques définis dans la revendication 1 (i) (b), ou tous les acides nucléiques définis dans la revendication 1 (i) (c) ; conjointement à tous les acides nucléiques définis dans la revendication 1 (ii) (a), ou à tous les acides nucléiques définis dans la revendication 1 (ii) (b), ou à tous les acides nucléiques définis dans la revendication 1 (ii) (c).

6. Procédé selon la revendication 5, dans lequel
b) dans une autre étape de détection, les fragments d'ADN obtenus à l'étape d'amplification a) sont détectés au moyen de sondes,
dans lequel les sondes utilisées à l'étape b) comprennent un ou plusieurs des acides nucléiques définis dans la revendication 2 (a) à (d).

7. Procédé selon l'une quelconque des revendications 5 et 6, qui permet également d'amplifier ou d'identifier dans le même échantillon le genre *Corynebacterium* et/ou le genre *Staphylococcus,* dans lequel
i) dans une deuxième étape d'amplification simultanée ou subséquente à la première étape d'amplification, l'ADN du genre *Corynebacterium* est amplifié avec des amorces conservées, et/ou
ii) dans une troisième étape d'amplification simultanée ou subséquente à la première étape d'amplification, l'ADN du genre *Staphylococcus* est amplifié avec des amorces conservées.

8. Procédé selon l'une quelconque des revendications 5 à 7, dans lequel les amorces utilisées à la deuxième étape d'amplification comprennent un ou plusieurs des acides nucléiques sélectionnés parmi
a) les SEQ ID n° 1, 3 ou 4 ;
b) les SEQ ID n° 46, 90, 91, 67, 68, 69, 70, 71, 73, 75, 72 ou 74 ;
c) une molécule d'acide nucléique qui s'hybride sous des conditions rigoureuses avec la molécule d'acide nucléique de l'un quelconque des points a) et b) ;
d) une molécule d'acide nucléique qui est au moins 90 % identique à la molécule d'acide nucléique du point a) ou b) ; ou
e) le complémentaire de la molécule d'acide nucléique de l'un quelconque des points a) et b).

9. Procédé selon l'une quelconque des revendications 5 à 8, dans lequel les amorces utilisées à la troisième étape d'amplification comprennent un ou plusieurs des acides nucléiques sélectionnés parmi
a) la SEQ ID N° 93 ;
b) les SEQ ID n° 94, 95, 96, 97, 98, 99, 100 ou 101 ;
c) une molécule d'acide nucléique qui s'hybride sous des conditions rigoureuses avec la molécule d'acide nucléique de l'un quelconque des points a) et b) ;
d) une molécule d'acide nucléique qui est au moins 90 % identique à la molécule d'acide nucléique du point a) ou b) ; ou
e) le complémentaire de la molécule d'acide nucléique de l'un quelconque des points a) et b).

10. Procédé selon l'une quelconque des revendications 5 à 9, permettant en outre l'amplification ou la détection d'un ou plusieurs des genres *Micrococcus, Kocuria, Kytococcus* et/ou *Nesterenkonia* ou d'une ou plusieurs espèces parmi un ou plusieurs de ces genres.

11. Procédé selon l'une quelconque des revendications 5 à 10, permettant en outre l'amplification ou la détection d'une ou plusieurs des espèces *Micrococcus luteus, Staphylococcus aureus* ou *Dermacoccus nishinomiyaensis*.
